(19) Europäisches Patentamt
European Patent Office
Office européen des brevets

(11) EP 1 978 102 B1

(12) **EUROPÄISCHE PATENTSCHRIFT**

(45) Veröffentlichungstag und Bekanntmachung des Hinweises auf die Patenterteilung:
**07.07.2010 Patentblatt 2010/27**

(51) Int Cl.:
*C12P 7/64* (2006.01) *C07C 69/587* (2006.01)
*C11C 1/04* (2006.01) *A23L 1/30* (2006.01)

(21) Anmeldenummer: **08005243.4**

(22) Anmeldetag: **20.03.2008**

(54) **Ein Gemisch enthaltend Fettsäureglyceride**

A mixture containing fatty acid glycerides

Un mélange comprenant des glycérides d'acides gras

(84) Benannte Vertragsstaaten:
**DE ES FR GB IT**

(30) Priorität: **02.04.2007 EP 07006845**
**02.04.2007 EP 07006846**

(43) Veröffentlichungstag der Anmeldung:
**08.10.2008 Patentblatt 2008/41**

(73) Patentinhaber: **Cognis IP Management GmbH**
**40589 Düsseldorf (DE)**

(72) Erfinder:
- **Schörken, Ulrich**
**40591 Düsseldorf (DE)**
- **Kempers, Peter**
**41189 Mönchengladbach (DE)**
- **Sander, Andreas**
**89257 Illertissen (DE)**
- **Horlacher, Peter**
**89287 Bellenberg (DE)**

(56) Entgegenhaltungen:
**WO-A-88/02775 WO-A-97/19601**
**WO-A-03/040091 KR-B1- 100 684 642**

- **HARALDSSON G G ET AL: "The Synthesis of Homogeneous Triglycerides of Eicosapentaenoic Acid and Docosahexaenoic Acid by Lipase" TETRAHEDRON, ELSEVIER SCIENCE PUBLISHERS, AMSTERDAM, NL, Bd. 51, Nr. 3, 16. Januar 1995 (1995-01-16), Seiten 941-952, XP004104914 ISSN: 0040-4020**
- **OKADA ET AL: "Production of n-3 polyunsaturated fatty acid concentrate from sardine oil by lipase-catalyzed hydrolysis" FOOD CHEMISTRY, ELSEVIER SCIENCE PUBLISHERS LTD, GB, Bd. 103, Nr. 4, 27. März 2007 (2007-03-27), Seiten 1411-1419, XP022003148 ISSN: 0308-8146**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 978 102 B1

Printed by Jouve, 75001 PARIS (FR)

## Beschreibung

Die vorliegende Erfindung betrifft ein Gemisch enthaltend Fettsäureglyceride, das einen hohen Anteil an PUFA-Acylresten und einen niedrigen Anteil an gesättigten Fettsäureacylresten enthält. Weiterhin betrifft die vorliegende Erfindung ein Verfahren, das es erlaubt, in einem Gemisch enthaltend Fettsäureglyceride (z. B. einem Fischöl) die PUFA-Acylreste anzureichern und gleichzeitig den Gehalt an gesättigten Fettsäureacylresten niedrig zu halten. Es handelt sich dabei um ein hydrolytisches Verfahren oder um eine Alkoholyse, bei dem die anzureichernden Fettsäureacylreste nicht oder nur langsam aus den Fettsäureglyceriden hydrolytisch oder alkohlytisch abgespalten werden, wobei das Verfahren in Gegenwart einer Lipase durchgeführt wird.

Im Sinne der vorliegenden Erfindung bedeutet Fettsäure eine beliebige gesättigte oder ungesättigte, verzweigte oder unverzweigte aliphatische Carbonsäure. Fettsäuren können gesättigt sein oder einfach, zweifach, dreifach etc. ungesättigt sein.

Fettsäuren als solche werden im Sinne der vorliegenden Erfindung auch als freie Fettsäuren bezeichnet. Im Vergleich dazu bedeutet im Sinne der vorliegenden Erfindung der Begriff Fettsäureacylrest den einbindigen Rest, der durch Entfernung des H-Atoms aus der COOH-Gruppe einer freien Fettsäure erhalten wird. Fettsäureacylreste kommen also zum Beispiel in freien Fettsäuren vor. Sie kommen auch in Estern von Fettsäuren, z. B. Estern mit Glycerin, den so genannten Glyceriden, vor. Ein Fettsäureglycerid ist ein Ester aus Glycerin und ein, zwei oder drei Fettsäureeinheiten. Ist nur eine OH-Gruppe des Glycerins mit einer Fettsäureeinheit verestert, spricht man von einem Monoglycerid. Sind zwei OH-Gruppen des Glycerins mit jeweils einer Fettsäureeinheit verestert, spricht man von einem Diglycerid. Sind alle drei OH-Gruppen des Glycerins mit jeweils einer Fettsäureeinheit verestert, spricht man von einem Triglycerid.

Im Sinne der vorliegenden Erfindung bedeutet PUFA eine Fettsäure, die mindestens zweifach ungesättigt ist. PUFA leitet sich ab vom englischen polyunsaturated fatty acid. In einer bevorzugten Ausführungsform der vorliegenden Erfindung bedeutet PUFA eine mindestens fünffach ungesättigte Fettsäure.

Eine omega-3-Fettsäure im Sinne der vorliegenden Erfindung ist eine mindestens zweifach (bevorzugt mindestens fünffach) ungesättigte Fettsäure. Sie gehört also zu den genannten PUFA. Eine omega-3-Fettsäure hat eine Doppelbindung zwischen dem dritten und dem vierten C-Atom gerechnet vom Methylende aus, wobei das Methyl-C-Atom als erstes C-Atom gezählt wird. Spezielle omega-3-Fettsäuren sind EPA ((all-Z)-5,8,11,14,17-Eicosapentaensäure) und DHA ((all-Z)-4,7,10,13,16,19-Docosahexaensäure).

Lipase A aus Candida antarctica im Sinne der vorliegenden Erfindung ist das Enzym wie beschrieben in J. Mol. Catal. B: Enzymatic 37 (2005), Seiten 36 bis 46. Lipase A aus Candida antarctica ist kommerziell erhältlich, z. B. als flüssige Lipasepräparation von der Firma Novozymes A/S unter dem Namen Novozym® 735.

PUFA-Glyceride, d. h. Glyceride mit einem hohen Anteil an PUFA-Acylresten an den insgesamt vorhandenen Fettsäureacylresten, werden nach dem Stand der Technik insbesondere nach einem der beiden folgenden Verfahren hergestellt:

1) Umesterung von Fischöl zu Ethylestern, destillative Anreicherung der PUFAs und Resynthese zu Glyceriden. Die Triglyceridsynthese wird dabei zumeist enzymatisch durchgeführt.
2) Selektive Hydrolyse von Fischölen zur Anreicherung der PUFAs in den Glyceriden und destillative Reinigung des PUFA-Glycerids. Die selektive Hydrolyse wird dabei zumeist enzymatisch durchgeführt.

Das Verfahren 1 (die enzymatische Triglyceridsynthese) ist zum Beispiel beschrieben in EP-A 0 528 844.

Das Verfahren 2 bzw. die Selektivität von Lipasen gegenüber PUFAs bei der Glyceridhydrolyse ist in mehreren Patentanmeldungen offenbart. Zu nennen sind insbesondere WO 97/19601, WO 95/24459, WO 96/37586, WO 96/37587, EP-A 0 741 183, WO 96/26287, WO 00/73254, WO 04/043894, WO 00/49117 **und** WO 91/16443.

Aus dem Stand der Technik ist folgendes über Enzymselektivitäten gegenüber PUFAs bekannt. Die meisten Lipasen und Phospholipasen haben eine negative Selektivität gegenüber PUFAs im Vergleich zu anderen Fettsäuren, die typischerweise in Pflanzen- und Fischölen enthalten sind. Mit negativer Selektivität ist gemeint, dass die Lipasen die anderen Fettsäurereste schneller aus Glyceriden hydrolytisch abspalten als die PUFA-Acylreste. Daher verläuft die enzymatische Anreicherung von PUFAs zumeist über eine Veränderung der anderen "nicht-PUFA"-Fettsäuren. Dies kann geschehen durch Veresterung, Umesterung oder Hydrolyse von Estern.

Negative Selektivitäten gegenüber PUFAs sind zum Beispiel beschrieben für Candida und Mucor Lipasen. Einige Enzyme, zum Beispiel solche isoliert aus Kaltwasserfischen, zeigen eine positive Selektivität gegenüber PUFAs

Lipase A aus Candida antarctica zeichnet sich durch einige besondere Eigenschaften aus: hohe Thermostabilität, eher eine sn2- als eine sn1,3-Spezifität (sn2- bzw. sn1,3-Spezifität ist die Spezifität der Lipasen bzgl. Glyceriden: snl,3 = bevorzugte Reaktion an den Außenpositionen von Glycerin, sn2 = bevorzugte Reaktion an der mittleren Position von Glycerin), hohe Selektivität gegenüber trans-Fettsäuren, eine Reaktion mit sterisch gehinderten Alkoholen und eine hohe Chemoselektivität zur N-Acylierung. Eine Zusammenfassung hierzu findet sich im folgenden Review-Artikel: J. Mol. Catal. B: Enzymatic 37 (2005), Seiten 36 bis 46. Eine Selektivität gegenüber gesättigten Fettsäuren ist hier nicht

beschrieben.

Lipasepräparationen aus Candida rugosa bzw. Aus Candida cylindracea sind ein Gemisch aus mindestens drei Enzymen, Lip 1, Lip 2 und Lip 3. Da kommerzielle Präparationen immer Mischungen der einzelnen Lipasen in variablen Anteilen sind, ist eine Charakterisierung der einzelnen Enzyme schwierig. Generell lässt sich feststellen, dass Lip 1 eine höhere Selektivität für lineare Alkohole hat, während Lip 2 und Lip 3 auch sterisch gehinderte Alkohole umsetzen können. Es ist sogar die Umsetzung von tertiären Alkoholen für Candida rugosa Lipase beschrieben worden. Eine Zusammenfassung hierzu findet sich in folgendem Review-Artikel: Biotechnology Advances 24 (2006), Seiten 180 bis 196.

Okada et al., Food chemistry, Bd. 103, Nr. 4, 27. März 2007, Seiten 1411-1419 offenbart ein Glyceridgemisch mit einem hohen Gehalt an mindestens dreifach ungesättigten Fettsäureresten und einem niedrigen Gehalt an gesättigten Fettsäureresten. Dieses Gemisch enthält hohe Mengen an EPA und DHA.

Yukihisa Tanaka et al., Journal of the American oil chemists society, Bd. 69, Nr. 12, 1. Dezember 1992, Seiten 1210-1214 offenbart die Anreicherung von DHA in Fischöl durch Lipase-katalysierte Hydrolyse. Es wird Lipase aus Candida cylindraceae verwendet.

WO 88/02775 offenbart die Hydrolyse von Olivenöl, welches mehrfach ungesättigte Fettsäurereste enthält in Gegenwart von Lipase A oder Lipase B aus Candida antarctica. Ferner wird die Selektivität von Lipase A gegenüber gesättigten Fettsäuren im Vergleich zu einfach- oder zweifach ungesättigten Fettsäuren beschrieben.

WO 03/040091 offenbart die Hydrolyse von Triglyceriden in Gegenwart von Lipasen.

Warwel S. et al., Biotechnology letters, Bd. 21, Nr. 5, 1999, Seiten 431-436 offenbart die Umesterung von Fettsäuremethylestern mit Butanol in Gegenwart von Lipasen.

WO 07/119811 offenbart die Alkoholyse von PUFA-haltigen Ölen oder Fetten in Gegenwart einer Lipase.

WO97/19601 offenbart ein zweistufiges Verfahren zur Herstellung einer Zusammensetzung auf Basis von Fischöl. Im ersten Schritt wird durch Hydrolyse von Fischöl mit einer Lipase ein Gemisch enthaltend Fettsäureglyceride erhalten, das einen hohen Anteil an PUFA-Acylresten und einen niedrigen Anteil an gesättigten Fettsäureacylresten enthält.

KR 100 684 642 offenbart ebenfalls ein Verfahren zur Herstellung eines Gemisches auf Basis von Fischöl, das einen hohen Anteil an PUFA-Acylresten und einen niedrigen Anteil an gesättigten Fettsäureacylresten enthält.

Fischöle bestehen im Wesentlichen aus Triglyceriden mit einer Mischung aus gesättigten, einfach und mehrfach ungesättigten Fettsäuren, dabei insbesondere einem hohen Anteil an 5-und 6-fach ungesättigten Fettsäuren, die als gesundheitsfördernder Nahrungsmittelzusatzstoff eingesetzt werden können. Da insbesondere die hochungesättigten Fettsäuren gesundheitsfördernd sind, ist es von Interesse diese anzureichern. Dies kann zum Beispiel durch ein selektives Entfernen der nicht hochungesättigten Fettsäuren aus den Triglyceriden geschehen. Zum Beispiel über eine selektive enzymatische Hydrolyse mit Hilfe von Lipasen.

Zu beachten ist hier allerdings, dass Partialglyceride (1-Mono-, 2-Mono- und 1,3-Diglyceride) höhere Schmelzpunkte aufweisen als die entsprechenden Triglyceridverbindungen (vgl. Tabelle 1).

**Tabelle 1: Vergleich der Schmelzpunkte verschiedener Glyceride [°C]**

| Fettsäure | 1-Mono | 2-Mono | 1,2-Di | 1,3-Di | Tri |
|---|---|---|---|---|---|
| C8 | 40 | 30 | | | 8 |
| C10 | 53 | 40 | | 45 | 32 |
| C12 | 63 | 51 | 41 | 58 | 46 |
| C14 | 71 | 61 | 56 | 66 | 58 |
| C16 | 77 | 69 | 63 | 73 | 66 |
| C18 | | 75 | 68 | 80 | 73 |
| C18:1 | 35 | | | 26 | 6 |
| C18:2 | 12 | | | - 3 | - 13 |

In Tabelle 1 wie auch im Folgenden wird folgende übliche Kurzbezeichnung für Fettsäuren oder Fettsäurereste (Acylreste) verwendet: Cx:y bezeichnet eine Fettsäure mit x C-Atomen und y Doppelbindungen.

Ebenfalls zu beachten ist, dass Fischöle einen deutlich höheren Anteil an gesättigten Fettsäuren besitzen als die meisten pflanzlichen Öle (vgl. Tabelle 2).

**Tabelle 2: Typische Fettsäurezusammensetzungen verschiedener Öle**

| Typische Zusammensetzung der Hauptbestandteile von Nahrungsmittelfetten (1 = Milchfett; 2 = Schweinetalg, 3 = Rindertalg, 4 = Sonnenblumenöl, 5 = Sojaöl, 6 = Olivenöl, 7 = Rapsöl, 8 = Palmöl, 9 = Sardinenöl, 10 = Thunfischöl, 11 = gehärtetes Pflanzenöl (Sonnenblume)) |
|---|

(fortgesetzt)

| Fettsäure | 1 | 2 | 3 | 4 | 5 | 6 | 7 | 8 | 9 | 10 | 11 |
|---|---|---|---|---|---|---|---|---|---|---|---|
| C4:0 | 3 | | | | | | | | | | |
| C6:0 - C10:0 | 5 | | | | | | | | | | |
| C12:0 | 3 | | | | | | | | | | |
| C14:0 | 10 | 3 | 4 | | | | 1 | 1 | 7 | 3 | |
| C16:0 | 33 | 24 | 30 | 4 | 10 | 11 | 4 | 39 | 18 | 21 | <10 |
| C16:1 | 4 | 3 | 3 | | | | | | 10 | 6 | |
| C18:0 | 9 | 8 | 22 | 4 | 4 | 2 | 1 | 5 | 3 | 6 | > 90 |
| C18:1 | 26 | 46 | 38 | 23 | 22 | 75 | 59 | 45 | 15 | 19 | |
| C 18:2 | 2 | 8 | 3 | 64 | 53 | 9 | 22 | 8 | | 2 | |
| C18:3 | | | 1 | | 8 | | 10 | | | 1 | |
| C20:5 | | | | | | | | | 17 | 7 | |
| C22:6 | | | | | | | | | 9 | 24 | |

Bleiben insbesondere die gesättigten Fettsäuren in Form von Partialglyceriden nach partieller Hydrolyse im Produktgemisch zurück, kann es leicht zu einer Präzipitation dieser Glyceride im angereicherten Fischöl-Glycerid führen. Gesättigte Partialglyceride haben erheblich höhere Schmelzpunkte als ungesättigte Triglyceride. Als Beispiel sei genannt der Schmelzpunkt von 1-Glycerinmonopalmitat mit 77 °C im Vergleich zu Trioleat mit 6 °C.

Bleiben solche gesättigten Glyceride, dabei insbesondere Partialglyceride im Produkt, verliert das Produkt seine Kältestabilität. Es kommt zu Ausflockungen im Produkt oder ggf. sogar zum Erstarren der Lipidmischung.

Der vorliegenden Erfindung liegt die Aufgabe zu Grunde, ein Gemisch bereit zu stellen, das Fettsäureglyceride enthält, wobei dieses Gemisch mindestens fünffach ungesättigte Fettsäureacylreste enthält und wobei dieses Gemisch einen niedrigen Schmelzpunkt haben soll, so dass es gut als Flüssigkeit verarbeitet werden kann, ohne dass durch Kristallisation des Gemisches oder von Teilen des Gemisches bei niedrigen Temperaturen Verarbeitungsprobleme entstehen. Bevorzugt soll das Gemisch einen möglichst hohen Anteil an mindestens fünffach ungesättigten Fettsäureacylresten enthalten, damit es aus diesem Grund besonders gut als Nahrungsmittelergänzungsstoff oder als Nahrungsmittelzusatzstoff eingesetzt werden kann.

Diese Aufgabe wird gelöst durch das folgende erfindungsgemäße Gemisch, das Gegenstand der vorliegenden Erfindung ist:

Ein Gemisch enthaltend

(A) optional mindestens ein Monoglycerid der Formel (I) oder (II),

(I)

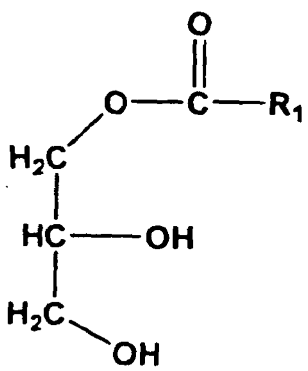

(II)

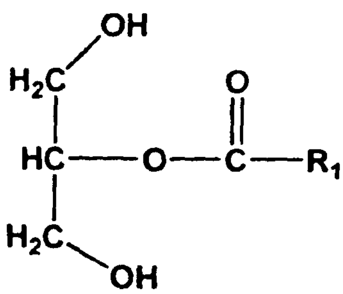

(B) mindestens ein Diglycerid der Formel (III) oder (IV) und

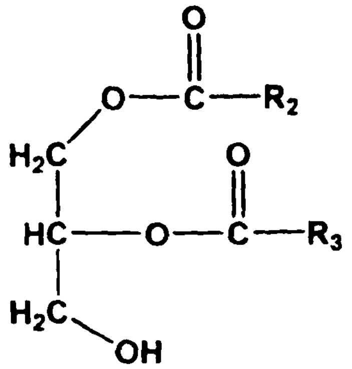

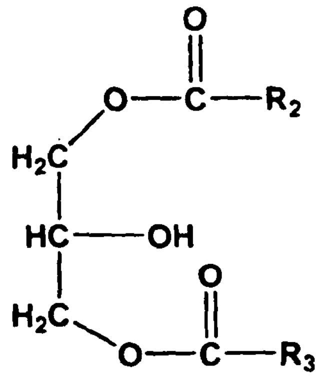

(C) mindestens ein Triglycerid der Formel (V),

(V)

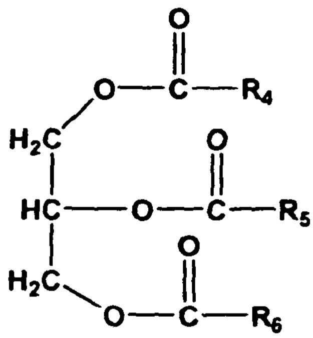

wobei die Reste $R_1$-CO-, $R_2$-CO-, $R_3$-CO-, $R_4$-CO-, $R_5$-CO- und $R_6$-CO- unabhängig voneinander ausgewählt werden aus der Gruppe bestehend aus einem gesättigten Fettsäureacylrest, einem einfach bis vierfach ungesättigten Fettsäureacylrest und einem mindestens fünffach ungesättigten Fettsäureacylrest,
und wobei die Summe der Massen aller in dem Gemisch vorhandenen mindestens fünffach ungesättigten Fettsäureacylreste, berechnet als freie Fettsäuren, bezogen auf die Summe der Massen aller in dem Gemisch vorhandenen Fettsäureacylreste (wiederum berechnet als freie Fettsäuren), mindestens 40 Gew.-% und höchstens 80 Gew.-%,
und wobei die Summe der Massen aller in dem Gemisch vorhandenen gesättigten Fettsäureacylreste berechnet als freie Fettsäure, bezogen auf die Summe der Massen aller in dem Gemisch vorhandenen Fettsäureacylreste (wiederum berechnet als freie Fettsäuren), höchstens

$$\frac{85-a}{3} \text{ Gew.-\%}$$

beträgt, wobei *a* die Summe der Massen aller in dem Gemisch vorhandenen mindestens fünffach ungesättigten Fettsäureacylreste, berechnet als freie Fettsäuren, bezogen auf die Summe der Massen aller in dem Gemisch vorhandenen Fettsäureacylreste (wiederum berechnet als freie Fettsäuren) in Gew.-% ist,
und wobei die Masse an Fettsäureacylresten der Stearinsäure, berechnet als freie Fettsäure, bezogen auf die Summe der Massen aller in dem Gemisch vorhandenen Fettsäureacylreste (wiederum berechnet als freie Fettsäuren), höchstens 2 Gew.-%beträgt,
und wobei der Gehalt an Triglyceriden bezogen auf alle Glyceride der Formeln I - V 50 bis 85 Gew.-% beträgt und zusätzlich die Bedingung erfüllt, dass der Gehalt an Triglyceriden bezogen auf alle Glyceride der Formeln I - V mindestens

$$\frac{410-5\cdot a}{3}\ \text{Gew.-\%}$$

beträgt, wobei a die oben definierte Bedeutung hat,
und wobei, der Gehalt an Diglyceriden bezogen auf alle Glyceride der Formeln I - V mindestens

$$100-3-b\ \text{Gew.-\%}$$

beträgt, wobei b der Gehalt an Triglyceriden bezogen auf alle Glyceride der Formeln I - V in Gew.-% ist.

Das erfindungsgemäße Gemisch enthält mindestens ein Diglycerid, mindestens ein Triglycerid und optional zusätzlich mindestens ein Monoglycerid.

Weiterhin sind folgende Gemische offenbart:

Das erfindungsgemäße Gemisch, wobei dieses Gemisch weiterhin enthält: mindestens 0,2 Gew.-% mindestens einer Komponente ausgewählt aus der Gruppe bestehend aus einem Phospholipid, Squalen, und Ceramid und/oder mindestens 20 ppm mindestens einer Komponente ausgewählt aus der Gruppe bestehend aus Vitamin A oder Provitamin A.

Das erfindungsgemäße Gemisch oder das Gemisch nach einer bereits genannten besonderen Ausführungsform, wobei dieses Gemisch weiterhin enthält: freie Fettsäuren und/oder Fettsäureethylester, wobei die Summe der Massen aller in dem Gemisch vorhandenen freien Fettsäuren oder Fettsäureethylester höchstens 2 Gew.-%, bezogen auf die Summe der Massen aller Monoglyceride plus der Massen aller Diglyceride plus der Massen aller Triglyceride plus der Massen aller freien Fettsäuren und/oder Fettsäureethylester in dem Gemisch, beträgt.

Das erfindungsgemäße Gemisch oder das Gemisch nach einer bereits genannten besonderen Ausführungsform, wobei die Summe der Massen aller in dem Gemisch vorhandenen Monoglyceride 0 bis 3 Gew.-%, bezogen auf die Summe der Massen aller Monoglyceride plus der Massen aller Diglyceride plus der Massen aller Triglyceride in dem Gemisch, beträgt.

Ein weiterer Gegenstand der vorliegenden Erfindung ist die Verwendung des erfindungsgemäßen Gemisches als Nahrungsergänzungsmittel oder als Nahrungsmittelzusatzstoff für die menschliche Ernährung oder als Fettermittel für Tierernährung.

Es wird auch die Verwendung als Futtermittel für die Tierernährung, insbesondere für eine Aquakultur mariner Organismen, insbesondere von Fischen und Crustaceen, offenbart.

Ein weiterer Gegenstand der vorliegenden Erfindung ist ein Verfahren zur Herstellung des erfindungsgemäßen Gemisches aus einem ersten Gemisch enthaltend

(A) optional mindestens ein Monoglycerid der Formel (I) oder (II),

(I)

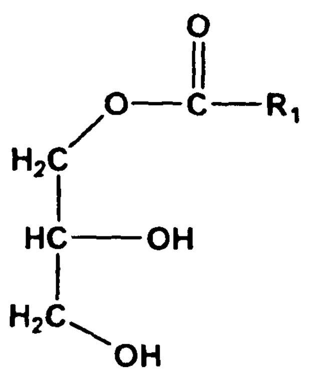

(II)

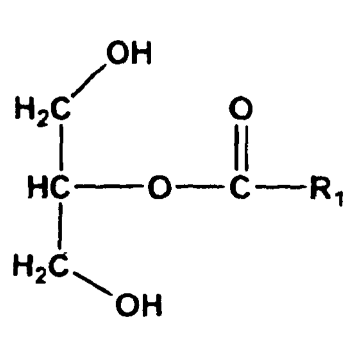

(B) optional mindestens ein Diglycerid der Formel (III) oder (IV) und

(III)

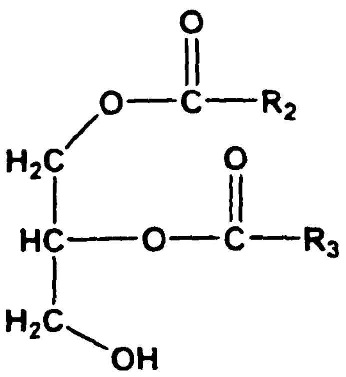

(IV)

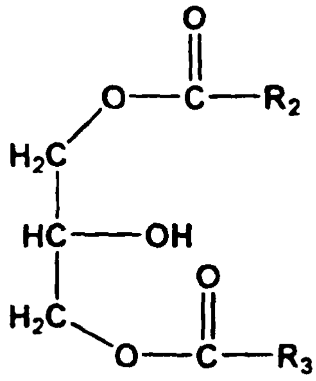

(C) mindestens ein Triglycerid der Formel (V),

(V)

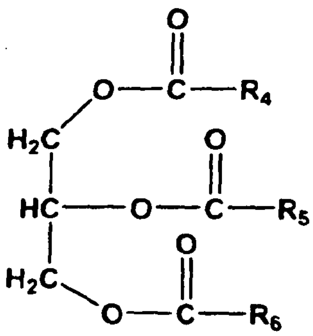

wobei die Reste $R_1$-CO-, $R_2$-CO-, $R_3$-CO-, $R_4$-CO-, $R_5$-CO- und $R_6$-CO- unabhängig voneinander ausgewählt werden aus der Gruppe bestehend aus einem gesättigten Fettsäureacylrest, einem einfach bis vierfach ungesättigten Fettsäureacylrest und einem mindestens fünffach ungesättigten Fettsäureacylrest,
und wobei die Summe der Massen aller in dem Gemisch vorhandenen mindestens fünffach ungesättigten Fettsäureacylreste, berechnet als freie Fettsäuren, bezogen auf die Summe der Massen aller in dem Gemisch vorhandenen Fettsäureacylreste (wiederum berechnet als freie Fettsäuren), mindestens um den Faktor 1,3 höher ist als im ersten Gemisch (Faktor A),
und wobei die Summe der Massen aller in dem Gemisch vorhandenen gesättigten Fettsäureacylreste berechnet als freie Fettsäure, bezogen auf die Summe der Massen aller in dem Gemisch vorhandenen Fettsäureacylreste (wiederum berechnet als freie Fettsäuren) mindestens um den Faktor 2 niedriger ist als im ersten Gemisch (Faktor B),
und wobei der Wert des Faktors A kleiner ist als der Wert des Faktors B,
und wobei der Gehalt des ersten Gemisches an Triglyceriden bezogen auf alle Glyceride der Formeln I - V mindestens 90 Gew.- % beträgt,
und wobei das erste Gemisch mindestens fünffach ungesättigten Fettsäureacylreste enthält, und wobei in einer bevorzugten Ausführungsform der vorliegenden Erfindung die Summe der Massen aller in dem ersten Gemisch vorhandenen mindestens fünffach ungesättigten Fettsäureacylreste, berechnet als freie Fettsäuren, bezogen auf die Summe der Massen aller in dem ersten Gemisch vorhandenen Fettsäureacylreste (wiederum berechnet als freie Fettsäuren), mindestens 15 Gew.-% beträgt,
und wobei das Verfahren die Umsetzung des ersten Gemisches mit Wasser (Hydrolyse) oder mit einem Alkohol mit 1 bis 4 C-Atomen (Alkoholyse) in Gegenwart von Lipase A aus Candida antarctica umfasst.

Die folgenden Verfahren sind besondere Ausführungsformen der vorliegenden Erfindung.

Das erfindungsgemäße Verfahren, wobei die Umsetzung des ersten Gemisches mit Wasser (Hydrolyse) oder mit einem Alkohol mit 1 bis 4 C-Atomen (Alkoholyse) in Gegenwart einer ersten, nicht regioselektiven Lipase mit einer erhöhten Spezifität für gesättigte Fettsäuren und einer zweiten, nicht regioselektiven Lipase mit einer erhöhten Spezifität

für einfach ungesättigten Fettsäuren im Vergleich zu allen ungesättigten Fettsäuren erfolgt, wobei die erste Lipase die Lipase A aus Candida antarctica ist, und wobei die zweite Lipase ausgewählt wird aus einer der Lipasen aus Candida rugosa oder Candida cylindracea.

Das erfindungsgemäße Verfahren oder das Verfahren nach einer bereits genannten besonderen Ausführungsform, wobei das erste Gemisch ausgewählt wird aus der Gruppe bestehend aus einem Fischöl, einem Öl von marinen Crustaceen , einem Öl von Mikroalgen, einem Öl von marinen Mikroorganismen und einem Öl marin lebender Säugetiere, wobei bevorzugt ein Öl mit einem Gehalt von mehr als 20 Gew.-% gebundenen mindestens fünffach ungesättigten Fettsäuren eingesetzt wird.

Das erfindungsgemäße Verfahren oder das Verfahren nach einer bereits genannten besonderen Ausführungsform, wobei die erste Lipase in freier oder immobilisierter Form eingesetzt wird.

Das erfindungsgemäße Verfahren oder das Verfahren nach einer bereits genannten besonderen Ausführungsform, wobei die zweite Lipase in freier oder immobilisierter Form eingesetzt wird.

Das erfindungsgemäße Verfahren oder das Verfahren nach einer bereits genannten besonderen Ausführungsform, wobei Wasser in einer Konzentration von 2 - 50 Gew.-% zur partiellen Hydrolyse zugegeben wird oder wobei Ethanol in einer Konzentration von 5 - 50 Gew.-% zugegeben wird, oder wobei eine Mischung von Wasser und Alkohol mit einer Gesamtkonzentration von 5 - 50 % bezogen auf den Ölanteil zugegeben wird.

Weiterhin werden folgende Ausführungsformen des Verfahrens offenbart:

Das erfindungsgemäße Verfahren oder das Verfahren nach einer bereits genannten besonderen Ausführungsform, wobei die Reaktion in einem Temperaturbereich von 20 - 80°C als Batchreaktion unter Rühren durchgeführt wird.

Das erfindungsgemäße Verfahren oder das Verfahren nach einer bereits genannten besonderen Ausführungsform, wobei die Reaktion in einem Temperaturbereich von 20 - 80°C als kontinuierliche Reaktion durchgeführt wird mit der wässrigen und oder alkoholischen Phase im Gleichstrom oder im Gegenstrom.

Das erfindungsgemäße Verfahren oder das Verfahren nach einer bereits genannten besonderen Ausführungsform, wobei dieses Verfahren weiterhin umfasst: eine Abtrennung der Wasser- und/oder alkoholhaltigen Phase über Separation, optional eine Abtrennung von immobilisiertem Enzym über Filtration, sowie eine Abtrennung der freigesetzten Fettsäuren bzw. Fettsäureester über Molekulardestillation, bei der das herzustellende Gemisch als Rückstand verbleibt. Eine besondere Ausführungsform ist dieses Verfahren, wobei die Destillation bei einer Temperatur unterhalb von 210 °C und bei einem Druck von maximal 0,3 mbar durchgeführt wird.

Das erfindungsgemäße Verfahren oder das Verfahren nach einer bereits genannten besonderen Ausführungsform, wobei dieses Verfahren weiterhin umfasst: eine Abtrennung der Wasser- und/oder alkoholhaltigen Phase über Separation, optional eine Abtrennung von immobilisiertem Enzym über Filtration, sowie eine erste Abtrennung der freigesetzten Fettsäuren bzw. Fettsäureester mit einer Kettenlänge kleiner C20 über Molekulardestillation und eine zweite Abtrennung langkettiger Fettsäuren bzw. Fettsäureester mit einer Kettenlänge größer C20 sowie Monoglyceriden über Molekulardestillation, bei der das herzustellende Gemisch als Rückstand verbleibt und ein monoglycerid-reiches Produkt als Destillat der zweiten Molekulardestillation erhalten wird. Eine besondere Ausführungsform ist dieses Verfahren, wobei die erste Destillation bei einer Temperatur unterhalb von 180 °C bei einem Druck von maximal 0,3 mbar durchgeführt wird, und bei dem die zweite Destillation bei einer Temperatur unterhalb von 210 °C bei einem Druck von maximal 0,3 mbar durchgeführt wird.

Das erfindungsgemäße Verfahren oder das Verfahren nach einer bereits genannten besonderen Ausführungsform, wobei das herzustellende Gemisch über Bleichung, insbesondere über Aktivkohlebehandlung gereinigt wird.

Das erfindungsgemäße Verfahren oder das Verfahren nach einer bereits genannten besonderen Ausführungsform, wobei das herzustellende Gemisch über Desodorierung, insbesondere über Desodorierung mit Wasserdampf oder Stickstoff bei einer Temperatur unterhalb von 200°C in Batch-Fahrweise oder auf kontinuierliche Weise gereinigt wird.

Das erfindungsgemäße Verfahren oder das Verfahren nach einer bereits genannten besonderen Ausführungsform, wobei das monoglycerid-reiche Produkt zur enzymatischen Resynthese mit immobilisierter Lipase B aus Candida antarctica bei einer Temperatur von 45 bis 80 °C und einem Druck von weniger als 30 mbar verwendet wird.

Das erfindungsgemäße Verfahren oder das Verfahren nach einer bereits genannten besonderen Ausführungsform, wobei i das glyceridreiche Gemisch über alkalische Umesterung mit Ethanol und einem Ethylat-Salz als Katalysator bei Temperaturen zwischen 40 °C und 120 °C in die entsprechenden Ethylester umgewandelt wird.

Das erfindungsgemäße Verfahren oder das Verfahren nach einer bereits genannten besonderen Ausführungsform, wobei der Ethylester über fraktionierte Destillation, bevorzugt über Molekulardestillation, bei Temperaturen unter 180 °C in eine Fraktion angereichert an fünf- und sechsfach ungesättigten Fettsäuren und eine abgereicherte Fraktion aufgespaltet wird.

Lipase A aus Candida antarctica hat folgende Vorteile:

1) Negative Selektivität für hochungesättigte Fettsäuren (PUFAs, insbesondere für EPA und DHA) und
2) Positive Selektivität für gesättigte Fettsäuren (insbesondere für Myristinsäure, Palmitinsäure und Stearinsäure).

Damit ist es möglich, angereicherte Glyceride mit hohem PUFA-Gehalt bei gleichzeitig niedrigem Gehalt an gesättigten Anteilen und insbesondere einem niedrigen Anteil an gesättigten Partialglyceriden bereit zu stellen.

Eine weiterer Vorteil des hier offenbarten Verfahrens besteht darin, dass es ein Hydrolyse- und Aufarbeitungsverfahren bereit stellt, bei dem ein Glyceridgemisch mit einer erhöhten PUFA-Konzentration und einer erniedrigten Konzentration an gesättigten Fettsäuren erhalten werden kann, wobei der Anteil an freien Säuren im Glyceridgemisch < 1 % und der Gehalt an Monoglyceriden < 5 % sein kann. Der niedrige Gehalt an freien Fettsäuren und Monoglyceriden ist von Bedeutung, da diese Verbindungen im Vergleich zu Di- und Triglyceriden negative sensorische Eigenschaften aufweisen (bitterer Geschmack) und in Form von gesättigten Fettsäuren leicht zu Produkttrübungen führen.

Es wurde gefunden, dass die Lipase A aus Candida antarctica eine deutlich negative Selektivität für PUFAs und eine positive Selektivität für gesättigte Fettsäuren aufweist. Kein weiteres kommerziell erhältliches Enzym mit ausgeprägter positiver Selektivität für gesättigte Fettsäuren wurde gefunden.

Es wurde ein Verfahren entwickelt zur Herstellung angereicherter PUFA-Glyceride. Dabei wird eine Mischung aus Fischöl und Wasser in Anwesenheit von Lipase A aus Candida antarctica gerührt und der Hydrolysegrad über die Entstehung der freien Säure kontrolliert. Die Lipase kann dabei in flüssiger oder immobilisierter Form eingesetzt werden. Nach Erreichen des gewünschten Hydrolysegrades wird das Wasser und das Enzym entfernt. Das Glyceridprodukt wird getrocknet und die freien Fettsäuren werden über eine Molekulardestillation aus dem Glyceridgemisch entfernt. Optional kann nachgeschaltet eine Bleichung und / oder Desodorierung des Produktes nach Standardmethoden erfolgen.

Die erfindungsgemäß hergestellten Glyceridgemische weisen eine deutliche Anreicherung der PUFAs und eine Abreicherung der gesättigten Fettsäuren bezogen auf die Ausgangszusammensetzung auf. Die so erhaltenen Glyceridgemische weisen eine bessere Kältestabilität auf als angereicherte PUFA-Glyceride, die mit einem negativ PUFA selektiven aber nicht selektiven Enzym für die gesättigten Fettsäuren (z B. Candida rugosa oder Candida cylindracea Lipase) hergestellt wurden.

Die Kombination von Lipase A aus Candida antarctica und Candida cylindracea Lipase bei der Hydrolyse führt ebenfall zu einem PUFA-Glycerid mit abgereichertern Gehalt an gesättigten Fettsäuren.

Lipase A aus Candida antarctica ist kommerziell erhältlich, zum Beispiel unter der Marke Novozym® 735, erhältlich von der Firma Novozymes A/S, Bagsvaerd, Dänemark.

Lipase aus Candida cylindracea ist kommerziell erhältlich, zum Beispiel unter der Marke Lipomod® 34, erhältlich von der Firma Biocatalysts Ltd., Pontypridd, UK.

Auch die übrigen Lipasen, die in den folgenden Beispielen verwendet wurden, sind kommerziell erhältlich. Die Lipase aus Geotrichum candidum wurde selbst hergestellt.

Im Folgenden bedeutet U "Unit" und ist eine Aktivitätsangabe für Enzyme. 1 U ist die Umsetzung von 1 μmol Substanz pro Minute unter bestimmten, definierten Reaktionsbedingungen.

Die Aktivitätsbestimmung für Lipase A aus Candida antarctica erfolgt wie folgt: (für Novozymes® A/S nach der Vorschrift für Novozym® 735): Es wird die Freisetzung von Buttersäure aus Glycerintributyrat bei 30°C und pH 7 bestimmt. Dazu wird eine 0,16 M Tributyrinlösung eingesetzt und Buttersäure wird unter konstantem pH-Wert mit NaOH titriert. 1 Unit entspricht der Aktivität, die 1 μmol Buttersäure aus Tributyrin pro Minute freisetzt.

Die Aktivitätsbestimmung für Lipase aus Candida cylindracea erfolgt wie folgt (nach Biocatalysts Ltd. Vorschrift für Lipomod® 34): Es wird die Freisetzung von Fettsäure aus Olivenöl in einer wässrigen Emulsion bestimmt unter konstantem pH-Wert durch NaOH Titration. 1 Unit entspricht der Aktivität, die 1 μmol Fettsäure aus Olivenöl pro Minute freisetzt.

Die Aktivitätsbestimmung für Lipase aus Candida rugosa erfogt wie folgt (nach Amano Inc. Vorschrift Lipase AY): Es wird die Freisetzung von Fettsäure aus Triglyceriden in einer wässrigen Triglycerid Emulsion bestimmt unter konstantem pH-Wert durch NaOH Titration. 1 Unit entspricht der Aktivität, die 1 μmol Fettsäure aus Triglycerid pro Minute freisetzt.

Im erfindungsgemäßen Verfahren kann Lipase A aus Candida antarctica alleine oder in Kombination mit einer zweiten Lipase mit negativer Selektivität für PUFAs, ausgewählt aus einer der Lipasen aus Candida rugosa oder Candida cylindracea, eingesetzt werden.

Die Lipase kann in freier oder immobilisierter Form eingesetzt werden.

Freie Form bedeutet dabei z. B. dass die Lipase direkt im wässrigen Anteil der Reaktionsmischung gelöst wird.

Immobilisierte Form bedeutet dabei z. B. eine Immobilisation auf Adsorberharze bzw. poröse Kunststoffe sowie eine Immobilisation auf ionenaustauscher. Alternativ kann auch eine Immobilisation über kovalente Bindungen an einen Träger erfolgen. Bevorzugt sind Adsorberharze (z.B. Polystyrene oder Polyacrylat Typen) sowie poröse Kunststoffe (z.B. Polypropylen).

Der Einsatz einer zweiten Lipase kann entweder gleichzeitig mit Lipase A aus Candida antarctica oder zeitversetzt als zweites Enzym zur Erhöhung des Spaltgrades erfolgen. Bevorzugt ist ein zeitversetzter Einsatz des zweiten Enzyms zur Erhöhung des Spaltgrades.

Im Folgenden werden besondere Ausführungsformen für das Verfahren genannt:

- Geeignete Öle als Ausgangsstoffe: Alle Fischöle sind geeignet, zusätzlich Öle aus PUFA produzierenden Mikroorganismen (zumeist Algen oder Pilze), marine Säugetiere und niedere marine Tiere (z.B. Krill). Bevorzugt sind Fischöle und mikrobielle Öle. Es können gereinigte Öle oder rohe Öle eingesetzt werden.

- Reaktionstemperatur: 20 - 80 °C, bevorzugt: 30 - 60 °C
- Wasseranteil bezogen auf Gesamtansatz: 2 - 80 %, bevorzugt: 5 - 50 %
- Weitere Bestandteile bei der enzymatischen Reaktion: keine nötig
- Enzymmenge Lipase A aus Candida antarctica: frei wählbar nach gewünschter Reaktionsdauer und gewünschtem Spaltgrad.
  Typische Werte:
  5 - 500 U freie Lipase / g Fischöl
  1 - 10 Gew.-% immobilisierte Lipase mit einem Gehalt an gebundener Lipase entsprechend 500 - 10.000 U freier Lipase pro g Träger
- 2. Enzym: Bevorzugt Candida cylindracea oder Candida rugosa Lipase
  Typische Werte für Enzymmengen:
  5 - 500 U freie Lipase / g Fischöl
  1 - 10 Gew% immobilisierte Lipase mit einem Gehalt an gebundener Lipase entsprechend 500 - 10.000 U freier Lipase pro g Träger
- Reaktionsdauer: abhängig von gewünschtem Spaltgrad und eingesetzter Enzymmenge; Typische Werte: 4 - 100 h
- Spaltgrad: frei einstellbar, sinnvoller Bereich 15 - 70 %;-Bevorzugter Bereich: 25 - 60 %

Im Folgenden werden besondere Ausführungsformen für das Verfahren in Bezug auf die Aufarbeitung des Produktes genannt:

- Entfernung der Fettsäuren über Destillation; typische Destillationsparameter: Vakuum < 1 mbar; T >/= 150 °C
- destillative Entfernung von Monoglyceride; typische Destillationsparameter: Vakuum < 1 mbar; T >/= 190 °C
- Raffination zur Entfernung von restlichen Fettsäuren
- Bleichung des Produktes (z.B. mit Bleicherden, Aktivkohlen)
- Geruchsentfernung aus dem Produkt (z.B. über Desodorierung mit Dampf oder Stickstoff, über Aktivkohlebehandlung
- Stabilisierung des Produktes mit Antioxidantien

Im Folgenden werden besondere Ausführungsformen für das Gemisch genannt:

- Summe EPA + DHA: > 35 %; bevorzugt > 40 %
- Summe PUFAs: > 40 %; bevorzugt > 50 %
- Summe omega-3 Fettsäuren: > 40 %; bevorzugt > 45 %
- Anteil freie Säure: < 10 %; bevorzugt < 2 %
- Anteil Monoglyceride: < 15 %; bevorzugt < 5 %
- Anteil Di- und Triglyceride: > 80 %; bevorzugt > 90 %
- Verhältnis Di- zu Triglyceriden: 1 : 100 bis 50:50
- Anteil an gebundenen gesättigten Fettsäuren: < 25 %; bevorzugt < 20 %

Die Kombination aus Lipase A aus Candida antarctica und einer anderen Lipase aus dem Genus Candida (bevorzugt entweder Lipase aus Candida rugosa oder Lipase aus Candida cylindracea oder eine Mischung aus diesen beiden Lipasen) hat folgende Vorteile:

3) Negative Selektivität für hochungesättigte Fettsäuren (PUFAs, insbesondere für EPA und DHA) und

4) Positive Selektivität für gesättigte Fettsäuren (insbesondere für Myristinsäure, Palmitinsäure und Stearinsäure).
5) eine synergistische Wirkung bei der selektiven Hydrolyse von Fischölen, gekennzeichnet durch:

- eine höhere Hydrolysegeschwindigkeit als die jeweiligen Enzyme alleine bei gleichem Enzymeinsatz auf Aktivitätsbasis
- einen höheren erreichbaren Spaltgrad pro Zeiteinheit. Damit sind unter anderem Gemische zugänglich, die einen sehr hohen PUFA-Gehalt haben. Wegen des dann erreichten hohen Hylolgegrades haben diese Gemische oft einen hohen Anteil an Diglyceriden, manchmal sogar mehr Diglyceride als Triglyceride
- eine vergleichbar gute negative Selektivität gegenüber omega-3 Fettsäuren als die jeweiligen Enzyme alleine

Damit ist es möglich, angereicherte Glyceride mit hohem PUFA-Gehalt bei gleichzeitig niedrigem Gehalt an gesättigten Anteilen und insbesondere einem niedrigen Anteil an gesättigten Partialglyceriden bereit zu stellen.

Es wurde gefunden, dass der gleichzeitige Einsatz von Candida antarctica A Lipase mit einer zweiten Lipase aus dem Genus Candida (Candida rugosa Lipase oder Candida cylindracea Lipase) eine synergistische Spaltwirkung besitzt. Es wurde eine höhere Hydrolysegeschwindigkeit erreicht und bei gleicher Enzymmenge konnte mit dem Enzymgemisch eine höhere Konzentration an omega 3 Fettsäuren in der Glyceridfraktion erzielt werden.

## Beispiele

### Beispiel 1: Screening nach positiver Selektivität für gesättigte Fettsäuren am Beispiel von Distelöl

10 g Distelöl und 5 g Wasser wurden mit verschiedenen Enzymen unter Rühren bei Raumtemperatur inkubiert. Proben der Ölphase wurden auf die Zusammensetzung der freigesetzten Fettsäuren hin analysiert. Die Summe an Palmitin- und Stearinsäure im Ausgangsöl betrug 10,0 %.

**Tabelle 3: Enzymscreening auf Basis Distelöl**

| Lipase aus Organismus | Summe Palmitin- und Stearinsäure |
|---|---|
| Alcaligenes sp | 17,5 % |
| Aspergillus niger | 23,3 % |
| Aspergillus oryzae | 22,7 % |
| Candida antarctica A | 29,0 % |
| Candida cylindracea | 7,6 % |
| Candida rugosa | 8,8 % |
| Geotrichum candidum | 3,6 % |
| Penicilium roqueforti | 15,4 % |
| Pseudomonas fluorescens | 18,3 % |
| Pseudomonas sp. | 11,2 % |
| Rhizopus javanicus | 18,9% |
| Rhizopus niveus | 17,7% |
| Rhizopus oryzae | 19,2 % |
| Thermomyces lanugenosus | 22,9 % |

Die meisten Enzyme zeigten eine Anreicherung an gesättigten Fettsäuren im Hydrolysat, was zumeist auf eine negative Selektivität gegenüber Trilinoleat zurückzuführen ist. Den höchsten Anteil an gesättigten Fettsäuren im Hydrolysat wies Lipase A aus Candida antarctica auf.

### Beispiel 2: Screening nach positiver Selektivität für gesättigte Fettsäuren am Beispiel von Rapsöl

10 g Rapsöl und 5 g Wasser wurden mit verschiedenen Enzymen unter Rühren bei Raumtemperatur inkubiert. Proben der Ölphase wurden auf die Zusammensetzung der freigesetzten Fettsäuren hin analysiert. Die Summe an Palmitin- und Stearinsäure im Ausgangsöl betrug 6,5 %.

**Tabelle 4: Enzymscreening auf Basis Rapsöl**

| Lipase aus Organismus | Summe Palmitin- und Stearinsäure |
|---|---|
| Aspergillus niger | 8,6 % |
| Aspergillus oryzae | 7,2 % |
| Candida antarctica A | 10,3 % |
| Candida cylindracea | 5,8 % |
| Candida rugosa | 5,6 % |
| Geotrichum candidum | 1,7 % |
| Mucor javanicus | 8,9 % |
| Penicilium roqueforti | 6,9 % |
| Porcine pancreas | 8,1 % |
| Pseudomonas cepacia | 8,0 % |
| Pseudomonas fluorescens | 7,7 % |
| Pseudomonas stutzeri | 7,4 % |
| Rhizomucor miehei | 7,8 % |
| Rhizopus javanicus | 8,4 % |
| Rhizopus niveus | 8,8 % % |
| Rhizopus oryzae | 8,1 % |
| Thermomyces lanugenosus | 8,1 % |

Einige der Enzyme zeigten eine leichte Anreicherung an gesättigten Fettsäuren im Hydrolysat, die deutlich schwächer ausgeprägt ist als bei der Hydrolyse von Distelöl. Den höchsten Anteil an gesättigten Fettsäuren im Hydrolysat wies auch hier Lipase A aus Candida antarctica auf.

**Beispiel 3: Screening nach negativer Selektivität für PUFAs am Beispiel von Makrelenöl**

10 g Makrelenöl und 10 g Wasser wurden in einen Kolben gegeben und unter Rühren auf 45°C bzw. 60 °C aufgeheizt. Verschiedene Enzyme wurden zu den Ansätzen gegeben in einer Menge von maximal 1 % an freiem Enzym (kommerzielle Enzympräparation) bzw. 3 % an immobilisiertem Enzym und die Ansätze wurden unter Rühren inkubiert. Während der Reaktion wurden Proben entnommen und der Umsatz wurde über Säurezahlmessung bestimmt. Ab einem Umsatz von > 40 % Hydrolysegrad wurde die Fettsäurezusammensetzung der freigesetzten Fettsäuren analysiert. Aus diesen Daten wurde rechnerisch der Gehalt an Glycerid gebundenen omega-3 Fettsäuren (darin enthalten hauptsächlich EPA und DHA) bestimmt. Enzyme, die keinen Umsatz von 40 % innerhalb von 24 h Reaktionszeit erreichten, wurden nicht weiter ausgewertet. Der Gehalt an omega-3 Fettsäuren im Ausgangsöl betrug 37,6 %.

**Tabelle 5: Enzymscreening auf Basis Makrelenöl**

| Enzym | Form | Temperatur | Umsatz | Omega-3 in Glycerid |
|---|---|---|---|---|
| Alcaligenes sp. | frei | 45 °C | 62 % | 51 % |
| Aspergillus niger | frei | 45 °C | < 40 % | |
| Candida antarctica A | frei | 45 °C | 44 % | 52 % |
| Candida antarctica A | immobilisiert | 60 °C | < 40 % | |
| Candida antarctica B | frei | 45 °C | < 40 % | |
| Candida antarctica B | immobilisiert | 60 °C | 43 % | 41 % |
| Candida cylindracea | frei | 45 °C | 43 % | 55 % |
| Candida rugosa | frei | 45 °C | 41 % | 55 % |
| Pseudomonas fluorescens | frei | 45 °C | 45 % | 47 % |
| Rhizomucor miehei | immobilisiert | 60 °C | < 40 % | |
| Rhizopus oryzae | frei | 45 °C | 43 % | 43 % |
| Rhizopus niveus | frei | 45 °C | < 40 % | |
| Thermomyces lanugenosus | frei | 45 °C | 61 % | 48 % |
| Thermomyces lanugenosus | immobilisiert | 45 °C | 60 % | 51 % |

Bei einer gleichzeitig guten Hydrolyseaktivität zeigten vor allem die Lipasen aus dem Organismus Candida

(Ausnahme Lipase B aus Candida antarctica) und Alcaligenes eine gute negative Selektivität gegenüber PUFAs. Eine leichte negative Selektivität gegenüber PUFAs wurde bei allen getesteten Enzymen beobachtet.

**Beispiel 4: Screening nach negativer Selektivität für PUFAs am Beispiel von Menhadenöl**

10 g Menhadenöl und 10 g Wasser wurden in einen Kolben gegeben und unter Rühren auf 45°C bzw. 60 °C aufgeheizt. Verschiedene Enzyme wurden zu den Ansätzen gegeben in einer Menge von maximal 1 % an freiem Enzym (kommerzielle Enzympräparation) bzw. 3 % an immobilisiertem Enzym und die Ansätze wurden unter Rühren inkubiert. Während der Reaktion wurden Proben entnommen und der Umsatz wurde über Säurezahlmessung bestimmt. Ab einem Umsatz von > 40 % Hydrolysegrad wurde die Fettsäurezusammensetzung der freigesetzten Fettsäuren analysiert. Aus diesen Daten wurde rechnerisch der Gehalt an Glycerid gebundenen omega-3 Fettsäuren (darin enthalten hauptsächlich EPA und DHA) bestimmt. Enzyme, die keinen Umsatz von 40 % innerhalb von 24 h Reaktionszeit erreichten, wurden nicht weiter ausgewertet. Der Gehalt an omega-3 Fettsäuren im Ausgangsöl betrug 38,0 %.

**Tabelle 6: Enzymscreening auf Basis Menhadenöl**

| Enzym | Form | Temp. | Umsatz | Omega-3 in Glycerid |
|---|---|---|---|---|
| Alcaligenes sp. | frei | 45 °C | 58 % | 53 % |
| Aspergillus niger | frei | 45 °C | < 40 % | |
| Candida antarctica A | frei | 45 °C | 52 % | 59 % |
| Candida antarctica A | immobilisiert | 60 °C | 47 % | 56 % |
| Candida antarctica B | frei | 45 °C | < 40 % | |
| Candida antarctica B | immobilisiert | 60 °C | 43 % | 45 % |
| Candida cylindracea | frei | 45 °C | 49 % | 61 % |
| Candida rugosa | frei | 45 °C | < 40 % | |
| Pseudomonas fluorescens | frei | 45 °C | 47 % | 49 % |
| Rhizomucor miehei | immobilisiert | 60 °C | < 40 % | |
| Rhizopus oryzae | frei | 45 °C | < 40 % | |
| Rhizopus niveus | frei | 45 °C | < 40 % | |
| Thermomyces lanugenosus | frei | 45 °C | 41 % | 49 % |
| Thermomyces lanugenosus | immobilisiert | 45 °C | 42 % | 50 % |

Bei einer gleichzeitig guten Hydrolyseaktivität zeigten vor allem die Lipasen aus dem Organismus Candida (Ausnahme Lipase B aus Candida antarctica) und Alcaligenes eine gute negative Selektivität gegenüber PUFAs. Eine leichte negative Selektivität gegenüber PUFAs wurde bei allen getesteten Enzymen beobachtet.

**Beispiel 5: Screening nach negativer Selektivität für PUFAs am Beispiel von Thunfischöl**

10 g Thunfischöl und 10 g Wasser wurden in einen Kolben gegeben und unter Rühren auf 45°C bzw. 60 °C aufgeheizt. Verschiedene Enzyme wurden zu den Ansätzen gegeben in einer Menge von maximal 1 % an freiem Enzym (kommerzielle Enzympräparation) bzw. 3 % an immobilisiertem Enzym und die Ansätze wurden unter Rühren inkubiert. Während der Reaktion wurden Proben entnommen und der Umsatz wurde über Säurezahlmessung bestimmt. Ab einem Umsatz von > 40 % Hydrolysegrad wurde die Fettsäurezusammensetzung der freigesetzten Fettsäuren analysiert. Aus diesen Daten wurde rechnerisch der Gehalt an Glycerid gebundenen omega-3 Fettsäuren (darin enthalten hauptsächlich EPA und DHA) bestimmt. Enzyme, die keinen Umsatz von 40 % innerhalb von 24 h Reaktionszeit erreichten, wurden nicht weiter ausgewertet. Der Gehalt an omega-3 Fettsäuren im Ausgangsöl betrug 39,6 %.

**Tabelle 7: Enzymscreening auf Basis Thunfischöl**

| Enzym | Form | Temp. | Umsatz | Omega-3 in Glycerid |
|---|---|---|---|---|
| Alcaligenes sp. | frei | 45 °C | 58 % | 53 % |
| Aspergillus niger | frei | 45 °C | < 40 % | |
| Candida antarctica A | frei | 45 °C | 63 % | 64 % |
| Candida antarctica A | immobilisiert | 60 °C | < 40 % | |
| Candida antarctica B | frei | 45 °C | < 40 % | |
| Candida antarctica B | immobilisiert | 60 °C | 43 % | 46 % |
| Candida cylindracea | frei | 45 °C | 53 % | 64 % |

(fortgesetzt)

| Enzym | Form | Temp. | Umsatz | Omega-3 in Glycerid |
|---|---|---|---|---|
| Candida rugosa | frei | 45 °C | 41 % | 57 % |
| Pseudomonas fluorescens | frei | 45 °C | 46 % | 50 % |
| Rhizomucor miehei | immobilisiert | 60 °C | < 40 % | |
| Rhizopus oryzae | frei | 45 °C | 54 % | 49 % |
| Rhizopus niveus | frei | 45 °C | < 40 % | |
| Thermomyces lanugenosus | frei | 45 °C | 61 % | 54 % |
| Thermomyces lanugenosus | immobilisiert | 45 °C | 55 % | 48 % |

Bei einer gleichzeitig guten Hydrolyseaktivität zeigten vor allem die Lipasen aus dem Organismus Candida (Ausnahme Lipase B aus Candida antarctica) eine gute negative Selektivität gegenüber PUFAs. Eine negative Selektivität gegenüber PUFAs wurde bei allen getesteten Enzymen beobachtet. Diese Selektivität ist bei DHA reichem Thunfischöl stärker ausgeprägt als bei den EPA reichen Fischölen.

**Beispiel 6: Selektive Hydrolyse von Fischöl 18/12 mit Lipase A aus Candida antarctica**

A) Enzymimmobilisation

40 g Accurel® MP 1000 (ein poröses Polypropylenpulver der Firma Membrana GmbH, Obernburg, Deutschland) wurden mit 400 g Aceton versetzt und für 5 min gerührt. Das Aceton wurde abfiltriert und das Accurel wurde mit Wasser gewaschen. Zum Accurel wurden 800 g Wasser und 40 g einer kommerziell erhältlichen Lipase A aus Candida antarctica (Novozym® 735) gegeben. Das Gemisch wurde für 20 h inkubiert und anschließend abfiltriert. Das Immobilisat wurde mit Wasser gewaschen und feucht für die Hydrolyse eingesetzt.

B) Reaktion + Aufarbeitung

800 g Fischöl 18/12 (ein Fischöl der Zusammensetzung wie angegeben in der folgenden Tabelle) und 800 g Wasser wurden in einen Doppelmantelreaktor gegeben und unter Rühren auf 45 °C aufgeheizt. Zum Ansatz wurde das feuchte Enzymimmobilisat gegeben und der Ansatz wurde bei konstanter Temperatur unter Rühren inkubiert. Die Säurezahl wurde stündlich gemessen. Bei einer Säurezahl von etwa 60 wurde der Rührer abgestellt. Nach Phasenseparation wurde die wässrige Phase abgetrennt und die Ölphase bei 80 °C im Rotationsverdampfer getrocknet. Die Säurezahl nach Separation und Trocknung betrug 67. Die Ölphase wurde an einer Kurzwegdestillation aufgearbeitet. Bei einer Temperatur von 200°C und einem Vakuum von 0,3 mbar wurden die freien Säuren über Kopf destilliert. Das Sumpfprodukt ist das angereicherte PUFA-Glycerid.

C) Produktanalyse

Das Produkt und vergleichend das Ausgangsmaterial wurde mit GPC (Gelpermeationschromatographie) (Glyceridverteilung); GC (Gaschromatographie) (Fettsäuremuster nach Methylierung) analysiert, sowie die Säurezahl gemessen. Die Ergebnisse sind in Tabelle 8 zusammengefasst.

**Tabelle 8: Fettsäureverteilung und Zusammensetzung im Glycerid und im Ausgangsprodukt**

| Fettsäure | Fischöl 18/12 [%] | PUFA-Glycerid [%] |
|---|---|---|
| 14:0 | 6,7 | 3,2 |
| 16:0 | 16,6 | 7,4 |
| 16:1 | 8,0 | 7,5 |
| 16:4 | 2,1 | 2,6 |
| 18:0 | 3,4 | 1,6 |
| 18:1 | 12,2 | 11,3 |
| 18:2 | 1,3 | 1,3 |
| 18:3 | 0,9 | 0,8 |
| 18:4 | 3,3 | 4,0 |
| 20:1 | 1,5 | 1,4 |
| 20:4 | 1,1 | 1,5 |

(fortgesetzt)

| Fettsäure | Fischöl 18/12 [%] | PUFA-Glycerid [%] |
|---|---|---|
| 20:5 | 18,4 | 24,2 |
| 22:5 | 2,2 | 2,7 |
| 22:6 | 12,8 | 18,5 |
| | | |
| Säurezahl | | 0,7 |
| | | |
| Triglycerid | > 95 | 72,6 |
| Diglycerid | | 27,1 |
| Monoglycerid | | 0,4 |
| Fettsäure | | < 0,5 |

**Beispiel 7: Selektive Hydrolyse von Fischöl 18/12 mit Lipase aus Candida cylindracea (Vergleichsbeispiel)**

A) Reaktion + Aufarbeitung

1000 g Fischöl 18/12 (ein Fischöl der Zusammensetzung wie angegeben in der folgenden Tabelle) und 250 g Wasser wurden in einen Doppelmantelreaktor gegeben und unter Rühren auf 45 °C aufgeheizt. Zum Ansatz wurden 750 mg einer kommerziell erhältlichen Lipase aus Candida cylindracea (Lipomod® 34) gegeben und der Ansatz wurde bei konstanter Temperatur unter Rühren inkubiert. Die Säurezahl wurde stündlich gemessen. Bei einer Säurezahl von etwa 60 wurde der Rührer abgestellt. Nach Phasenseparation wurde die wässrige Phase abgetrennt und die Ölphase bei 80 °C im Rotationsverdampfer getrocknet. Die Säurezahl nach Separation und Trocknung betrug 60. Die Ölphase wurde an einer Kurzwegdestillation aufgearbeitet. Bei einer Temperatur von 200 °C und einem Vakuum von 0,3 mbar wurden die freien Säuren Säuren über Kopf destilliert. Das Sumpfprodukt ist das angereicherte PUFA-Glycerid.

B) Produktanalyse

Das Produkt und vergleichend das Ausgangsmaterial wurde mit GPC (Glyceridverteilung); GC (Fettsäuremuster nach Methylierung) analysiert sowie die Säurezahl gemessen. Die Ergebnisse sind in Tabelle 9 zusammengefasst.

**Tabelle 9: Fettsäureverteilung und Zusammensetzung im Glycerid und im Ausgangsprodukt**

| Fettsäure | Fischöl 18/12 [%] | PUFA-Glycerid [%] |
|---|---|---|
| 14:0 | 6,7 | 6,0 |
| 16:0 | 16,6 | 13,2 |
| 16:1 | 8,0 | 3,8 |
| 16:4 | 2,1 | 2,5 |
| 18:0 | 3,4 | 3,4 |
| 18:1 | 12,2 | 7,4 |
| 18:2 | 1,3 | 0,7 |
| 18:3 | 0,9 | 0,5 |
| 18:4 | 3,3 | 4,1 |
| 20:1 | 1,5 | 1,7 |
| 20:4 | 1,1 | 1,4 |
| 20:5 | 18,4 | 22,6 |
| 22:5 | 2,2 | 2,9 |
| 22:6 | 12,8 | 18,0 |
| | | |
| Säurezahl | | 1,4 |
| | | |
| Triglycerid | > 95 | 78,0 |
| Diglycerid | | 20,5 |
| Monoglycerid | | 1,2 |
| Fettsäure | | < 1,0 |

**Beispiel 8: Vergleich der Produkte aus den Beispielen 6 + 7**

| Beispiel 6: | Ausgangsmaterial | Produkt | An-/Abreicherung |
|---|---|---|---|
| Summe omega 3 Fettsäuren | 37,6 % | 50,2 % | 1,33 |
| Summe fünf- und sechsfach ungesättigte Fettsäuren | 33,4 % | 45,4 % | 1,36 |
| Summe gesättigte Fettsäuren (C14:0; C16:0 und C 18:0) | 26,7 % | 12,2 % | 2,19 |

| Beispiel 7: | Ausgangsmaterial | Produkt | An-/Abreicherung |
|---|---|---|---|
| Summe omega 3 Fettsäuren | 37,6 % | 48,1 % | 1,28 |
| Summe fünf- und sechsfach ungesättigte Fettsäuren | 33,4 % | 43,5 % | 1,3 |
| Summe gesättigte Fettsäuren (C14:0; C16:0 und C18:0) | 26,7 % | 22,6 % | 1,18 |

Es ist deutlich zu erkennen, dass bei einer vergleichbaren omega-3 PUFA Anreicherung und einer vergleichbaren Glyceridverteilung beim Einsatz von Lipase A aus Candida antarctica (Beispiel 6) eine deutlich erniedrigte Konzentration an gesättigten Fettsäuren im Produkt erreicht wird als bei Einsatz von Candida cylindracea (Beispiel 7).
Dies lässt sich auch über den entsprechenden Anreicherungs- bzw. Abreicherungsfaktor vergleichen, der wie folgt berechnet wurde:
Anreicherungsfaktor omega-3 Fettsäuren = Produkt [%] / Ausgangsmaterial [%]
Abreicherungsfaktor gesättigte Fettsäuren = Ausgangsmaterial [%] / Produkt [%]

**Beispiel 9: Selektive Hydrolyse von Fischöl 18/12 mit Lipase A aus Candida antarctica und aus Candida cylindracea**

1000 g Fischöl 18/12 (ein Fischöl der Zusammensetzung wie angegeben in der folgenden Tabelle) und 500 g Wasser wurden in einem Reaktor unter Rühren auf 45 °C temperiert. Dazu wurden 25 g immobilisierte Lipasse aus Candida A (analog zu Beispiel 6) gegeben und der Ansatz wurde über Nacht inkubiert. Dann wurden 1 g Lipase aus Candida cylindracea (Lipomod® 34) zum Ansatz gegeben und der Ansatz wurde inkubiert, bis eine Säurezahl von > 100 erreicht war. Das Rohprodukt wies eine Säurezahl von 112 auf. Die Aufarbeitung des Reaktionsansatzes erfolgt analog zu den Beispielen 6 und 7. Die Ergebnisse sind in Tabelle 10 zusammengefasst.

**Tabelle 10: Fettsäureverteilung und Zusammensetzung im Glycerid und im Ausgangsprodukt**

| Fettsäure | Fischöl 18/12 [%] | PUFA-Glycerid [%] |
|---|---|---|
| 14:0 | 6,7 | 2,6 |
| 16:0 | 16,6 | 7,8 |
| 16:1 | 8,0 | 4,0 |
| 16:4 | 2,1 | 2,4 |
| 18:0 | 3,4 | 2,1 |
| 18:1 | 12,2 | 8,1 |
| 18:2 | 1,3 | 1,0 |
| 18:3 | 0,9 | 0,6 |
| 18:4 | 3,3 | 4,3 |
| 20:1 | 1,5 | 1,3 |
| 20:4 | 1,1 | 1,7 |
| 20:5 | 18,4 | 26,5 |
| 22:5 | 2,2 | 3,2 |
| 22:6 | 12,8 | 24,3 |

**Beispiel 10: Selektive Hydrolyse von Brudy Algatrium Thunfischöl mit Lipase A aus Candida antarctica und**

**aus Candida cylindracea**

Die Reaktion wurde analog zu Beispiel 9 mit 1000 g Brudy Algatrium DHA 18 Thunfischöl (ein Fischöl gewonnen aus Thunfischen mit einer Fettsäurezusammensetzung wie unten spezifiziert von der Firma Proyecto Empresarial Brudy, Barcelona, Spanien mit dem Handelsnamen Algatrium® DHA 18) durchgeführt. Das Rohprodukt wies eine Säurezahl von 104 auf. Die Ergebnisse sind in Tabelle 11 zusammengefasst.

**Tabelle 11: Fettsäureverteilung und Zusammensetzung im Glycerid und im Ausgangsprodukt**

| Fettsäure | Fischöl DHA 18 [%] | PUFA-Glycerid [%] |
|---|---|---|
| 14:0 | 4,1 | 1,6 |
| 16:0 | 18,3 | 5,4 |
| 16:1 | 5,3 | 2,7 |
| 18:0 | 4,9 | 2,1 |
| 18:1 | 17,8 | 10,2 |
| 18:2 | 1,8 | 1,1 |
| 18:3 | 0,7 | |
| 18:4 | 1,2 | 1,7 |
| 20:1 | 2,4 | 2,2 |
| 20:4 | 1,8 | 3,0 |
| 20:5 | 8,5 | 12,2 |
| 22:5 | 1,7 | 2,7 |
| 22:6 | 21,6 | 44,3 |

**Beispiel 11: Selektive Hydrolyse von Brudy Algatrium Thunfischöl mit Lipase aus Candida cylindracea**

800 g Brudy Algatrium DHA 18 Thunfischöl und 200 g Wasser wurden in einem Reaktor unter Rühren auf 45°C temperiert. Dazu wurden 2 g Lipase aus Candida cylindracea (Lipomod® 34) gegeben und der Ansatz wurde inkubiert, bis eine Säurezahl von > 100 erreicht war. Das Rohprodukt wies eine Säurezahl von 122 auf. Die Aufarbeitung des Reaktionsansatzes erfolgt analog zu den Beispielen 6 und 7. Die Ergebnisse sind in Tabelle 12 zusammengefasst.

**Tabelle 12: Fettsäureverteilung und Zusammensetzung im Glycerid und im Ausgangsprodukt**

| Fettsäure | Fischöl DHA 18 [%] | PUFA-Glycerid [%] |
|---|---|---|
| 14:0 | 4,1 | 2,3 |
| 16:0 | 18,3 | 10,4 |
| 16:1 | 5,3 | 3,0 |
| 18:0 | 4,9 | 3,0 |
| 18:1 | 17,8 | 10,4 |
| 18:2 | 1,8 | 1,0 |
| 18:3 | 0,7 | 0,4 |
| 18:4 | 1,2 | 1,2 |
| 20:1 | 2,4 | 1,9 |
| 20:4 | 1,8 | 2,4 |
| 20:5 | 8,5 | 7,4 |
| 22:5 | 1,7 | 2,6 |
| 22:6 | 21,6 | 45,2 |

**Beispiel 12: Selektive Hydrolyse von Fischöl Lamotte Typ 170 mit Lipase aus Candida cylindracea**

Die Reaktion wurde analog zu Beispiel 7 mit 800 g Fischöl Lamotte Typ 170 (ein Fischöl der Firma Henry Lamotte GmbH, Bremen mit dem Handelsnamen Typ 170 mit unten aufgelisteter Fettsäurezusammensetzung) durchgeführt. Das Rohprodukt wies eine Säurezahl von 111 auf. Die Ergebnisse sind in Tabelle 13 zusammengefasst.

**Tabelle 13: Fettsäureverteilung und Zusammensetzung im Glycerid und im Ausgangsprodukt**

| Fettsäure | Fischöl Typ 170 [%] | PUFA-Glycerid [%] |
|---|---|---|
| 14:0 | 5,0 | 2,5 |
| 16:0 | 18,2 | 9,4 |
| 16:1 | 6,1 | 3,0 |
| 18:0 | 4,6 | 2,8 |
| 18:1 | 16,8 | 9,2 |
| 18:2 | 1,5 | 0,8 |
| 18:3 | 0,7 | 0,4 |
| 18:4 | 1,4 | 1,5 |
| 20:1 | 2,4 | 2,3 |
| 20:4 | 1,6 | 2,1 |
| 20:5 | 10,5 | 9,9 |
| 22:5 | 1,9 | 3,1 |
| 22:6 | 19,3 | 38,8 |

**Beispiel 13: Vergleich der Produkte aus den Beispielen 9 bis 12: Selektivität gegenüber gesättigten Fettsäuren**

| Beispiel 9: | Ausgangsmaterial | Produkt | An-/Abreicherung |
|---|---|---|---|
| Summe omega 3 Fettsäuren | 37,6 % | 58,9 % | 1,57 |
| Summe gesättigte Fettsäuren (14, 16, 18) | 26,7 % | 12,5 % | 2,14 |
| Summe gesättigte Fettsäuren im Destillat | | 40,6 % | |

| Beispiel 10: | Ausgangsmaterial | Produkt | |
|---|---|---|---|
| Summe omega 3 Fettsäuren | 33,7 % | 61,4 % | 1,82 |
| Summe gesättigte Fettsäuren (14, 16, 18) | 27,3 % | 9,1 % | 3,0 |
| Summe gesättigte Fettsäuren im Destillat | 44,5 % | | |

| Beispiel 11: | Ausgangsmaterial | Produkt | |
|---|---|---|---|
| Summe omega 3 Fettsäuren | 33,7 % | 57,2 % | 1,7 |
| Summe gesättigte Fettsäuren (14, 16, 18) | 27,3 % | 15,7 % | 1,74 |
| Summe gesättigte Fettsäuren im Destillat | | 31,3 % | |

| Beispiel 12: | Ausgangsmaterial | Produkt | |
|---|---|---|---|
| Summe omega 3 Fettsäuren | 33,8 % | 54,1 % | 1,6 |
| Summe gesättigte Fettsäuren (14, 16, 18) | 27,8 % | 14,7 % | 1,89 |
| Summe gesättigte Fettsäuren im Destillat | | 35,1 % | |

Als gesättigte Fettsäuren wurde die Summe an C14:0, C16:0 und C18:0 gesetzt.

Es ist deutlich zu erkennen, dass bei einer vergleichbaren omega-3-PUFA Anreicherung beim Einsatz einer Mischung von Lipase A aus Candida antarctica und Lipase aus Candida cvylindracea (Beipiele 9 und 10) eine deutlich erniedrigte Konzentration an gesättigten Fettsäuren im Produkt erreicht wurde, verglichen zum alleinigen Einsatz von Lipase aus Candida cylindracea (Beispiele 11 und 12). Der Selektivitätsunterschied ist insbesondere am Anteil der abgespaltenen Fettsäuren (Destillat) zu erkennen. Dies lässt sich auch über den entsprechenden Anreicherungs- bzw Abreicherungsfaktor vergleichen, der wie folgt berechnet wurde: Anreicherungsfaktor omega-3 Fettsäuren = Produkt [%] / Ausgangsmaterial [%] Abreicherungsfaktor gesättigte Fettsäuren = Ausgangsmaterial [%] / Produkt [%]

**Beispiel 14: Vergleich der Produkte aus den Beispielen 9 bis 12: Selektivität gegenüber PUFAs**

| Beispiel 9: | Ausgangsmaterial | Produkt |
|---|---|---|
| Summe fünf- und sechsfach ungesättigte Fettsäuren | 33,4 % | 54 % |
| Summe Eicosapentaensäure (EPA, C20:5) | 18,4 % | 26,5 % |
| Summe Docosahexaensäure (DHA, C22:6) | 12,8 % | 24,3 % |

| Beispiel 10: | Ausgangsmaterial | Produkt |
|---|---|---|
| Summe fünf- und sechsfach ungesättigte Fettsäuren | 31,8 % | 59,2 % |
| Summe Eicosapentaensäure (EPA, C20:5) | 8,5 % | 12,2 % |
| Summe Docosahexaensäure (DHA, C22:6) | 21,6 % | 44,3 % |

| Beispiel 11: | Ausgangsmaterial | Produkt |
|---|---|---|
| Summe fünf- und sechsfach ungesättigte Fettsäuren | 31,8 % | 55,2 % |
| Summe Eicosapentaensäure (EPA, C20:5) | 8,5 % | 7,4 % |
| Summe Docosahexaensäure (DHA, C22:6) | 21,6 % | 45,2 % |

| Beispiel 12: | Ausgangsmaterial | Produkt |
|---|---|---|
| Summe fünf- und sechsfach ungesättigte Fettsäuren | 31,7 % | 51,8 % |
| Summe Eicosapentaensäure (EPA, C20:5) | 10,5 % | 9,9 % |
| Summe Docosahexaensäure (DHA, C22:6) | 19,3 % | 38,8 % |

Es ist deutlich zu erkennen, dass die omega-3-PUFA Anreicherung beim Einsatz einer Mischung von Lipase A aus Candida antarctica und Lipase aus Candida cylindracea größer war als beim alleinigen Einsatz von Candida cylindracea Lipase. Der Selektivitätsunterschied ist insbesondere auf die höhere Selektivität der Lipase A aus Candida antarctica gegenüber EPA zurückzuführen, der bei Spaltgraden im Öl von > 50 % signifikant wird.

**Beispiel 15: Selektive Hydrolyse von Napro 18/12 Fischöl mit Candida antarctica A und Candida cylindracea Lipase**

3500 g Fischöl 18/12 (Napro Pharma) und 1750 g Wasser wurden in einem Reaktor unter Rühren auf 45 °C temperiert. Dazu wurden 87,5 g immobilisierte Candida A Lipase (analog zu Beispiel 6) gegeben und der Ansatz wird über Nacht inkubiert. Dann wurden 2,5 g Candida cylindracea Lipase (Lipomod 34) zum Ansatz gegeben und der Ansatz wurde 4 h inkubiert. Das Rohprodukt wies eine Säurezahl von 96 auf.
Nach Phasenseparation wurde die wässrige Phase abgetrennt und die Ölphase bei 80 °C im Rotationsverdampfer getrocknet. Die Ölphase wurde an einer Kurzwegdestillation aufgearbeitet. Bei einer Temperatur von 190 °C und einem Vakuum von 0,3 mbar wurden die freien Säuren Säuren über Kopf destilliert. Die Destillation wurde zweimal durchgeführt. Das Sumpfprodukt war das angereicherte PUFA-Glycerid. Die Muster wurden gaschromatographisch auf ihre Fettsäureverteilung hin untersucht und mittels GPC auf ihre Glyceridzusammensetzung.

**Tabelle 14: PUFA Anreicherung auf Basis Napro 18/12 Fischöl**

| Fettsäure | Fischöl 18/12 [%] | PUFA-Glycerid [%] | Ab-/Anreicherung |
|---|---|---|---|
| 14:0 | 6,7 | 2,5 | |
| 16:0 | 16,6 | 5,9 | |
| 16:1 | 8,0 | 4,4 | |
| 16:4 | 2,1 | 3,5 | |
| 18:0 | 3,4 | 1,4 | |
| 18:1 | 12,2 | 7,7 | |
| 18:2 | 1,3 | 0,9 | |
| 18:3 | 0,9 | 0,5 | |
| 18:4 | 3,3 | 4,5 | |
| 20:1 | 1,5 | 0,9 | |

(fortgesetzt)

| Fettsäure | Fischöl 18/12 [%] | PUFA-Glycerid [%] | Ab-/Anreicherung |
|---|---|---|---|
| 20:4 | 1,1 | 1,7 | |
| 20:5 | 18,4 | 28,9 | |
| 22:5 | 2,2 | 3,5 | |
| 22:6 | 12,8 | 22,9 | |
| | | | |
| Summe SAFA (14,16,18) | 26,7 | 9,8 | 2,7 |
| | | | |
| Summe MUFA | 21,7 | 13 | 1,7 |
| Summe PUFA | 40,8 | 65,5 | 1,6 |
| Summe omega3 | 37,6 | 60,3 | 1,6 |
| Summe 5 und 6 fach Ungesättigte Fettsäuren | 33,4 | 55,3 | 1,7 |
| Zugeordnet | 90,5 | 89,2 | |
| | | | |
| Triglycerid | > 95 | 66,5 | |
| Diglycerid | | 31,1 | |
| Monoglycerid | | 2,2 | |
| Fettsäure | | 0,3 | |

SAFA bedeutet gesättigte Fettsäuren (abgeleitet vom englischen saturated fatty acid) und MUFA bedeutet einfach ungesättigte Fettsäuren (abgeleitet vom englischen monounsaturated fatty acid). Die Anreicherungsfaktoren werden analog zu Beispiel 13 bestimmt.

**Beispiel 16: Alkalische Hydrolyse der angereicherten Glyceride aus Beispiel 16**

1250 g getrocknete Glyceridfraktion wurden mit 500 g Ethanol in einen Reaktor ausgerüstet mit Rührer, Tropftrichter und Rückflusskühler vorgelegt und unter Rühren aufgeheizt, bis sich Ethanol Rückfluss einstellt. Über 1 h wurden dann 1250 g einer Kaliumhydroxid-Lösung bestehend aus 937,5 g Wasser und 312,5 g KOH zugetropft und das Gemisch wurde für weitere 3 h unter Rückfluss gehalten. Es wurden weitere 875 g Wasser in den Reaktor gegeben und anschließend wurden 575 g konzentrierte Phosphorsäure über einen Zeitraum von 30 min zudosiert. Zur Neutralisation wurden weitere 30 min nachgerührt, dann wurde der Rührer abgestellt. Nach Phasenseparation wurde die wässrige Phase über ein Bodenablassventil abgetrennt. Die Ölphase wurde zweimal mit 2500 g Wasser gewaschen und anschließend am Rotationsverdampfer getrocknet.
**Ergebnis:** Die neutralisierten und getrockneten Fettsäuren hatten eine Säurezahl von 192. Das Fettsäurespektrum entsprach dem der eingesetzten angereicherten Glyceride.

**Beispiel 17: Alkalische Umesterung der angereicherten Glyceride aus Beispiel 16 und destillative PUFA Anreicherung der Ethylesterfraktion**

500 g getrocknete Glyceridfraktion wurden mit 200 g Ethanol in einen Reaktor ausgerüstet mit Rührer, Tropftrichter und Rückflusskühler vorgelegt und unter Rühren und Stickstoffbegasung auf 60 °C aufgeheizt. 20 g 21 Gew.-% Natriumethylat in Ethanol wurden zugegeben und die Reaktion wurde für 6 h bei 60 °C gehalten. Nach 2 h wurden weitere 10 g Natriumethylat-Lösung zugegeben. Nach 6 h wurde die Reaktion mit 2 %iger Zitronensäure versetzt, bis der pH-Wert des Reaktionsgemisches unterhalb von pH 6 lag. Der Rührer wurde ausgestellt und nach Phasenseparation wurde die Ölphase von der wässrigen Phase abgetrennt und am Rotationsverdampfer getrocknet. Die Ölphase wurde an einer Kurzwegdestillation aufgearbeitet. Bei einer Temperatur von 145°C und einem Vakuum von 0,3 mbar wurde ein Teil der küzerkettigen Fettsäuren (Kettenlänge </= C18) abdestilliert. Das Sumpfprodukt wurde dann bei einer Temperatur von 185 °C und einem Vakuum von 0,3 mbar komplett über Kopf destilliert. Dieses Destillat wurde gaschromatographisch auf seine Fettsäureverteilung hin untersucht.

Tabelle 15: PUFA Anreicherung auf Basis Napro 18/12 Fischöl

| Fettsäure | PUFA-Glycerid [%] Aus Beispiel 16 | Ethylester [%] destillativ angereichert |
|---|---|---|
| 14:0 | 2,5 | 0,4 |
| 16:0 | 5,9 | 2,8 |
| 16:1 | 4,4 | 1,9 |
| 17:0 | 3,5 | 1,4 |
| 18:0 | 1,4 | 1,3 |
| 18:1 | 7,7 | 7,0 |
| 18:2 | 0,9 | 0,8 |
| 18:3 | 0,5 | 0,6 |
| 18:4 | 4,5 | 3,8 |
| 20:1 | 0,9 | 1,2 |
| 20:4 | 1,7 | 2,0 |
| 20:5 | 28,9 | 33,8 |
| 22:5 | 3,5 | 4,8 |
| 22:6 | 22,9 | 29,7 |
| | | |
| Summe SAFA | 13,3 | 5,9 |
| Summe MUFA | 13,1 | 10,1 |
| Summe PUFA | 63,1 | 75,5 |
| Summe omega3 | 60,4 | 72,7 |
| Zugeordnet | 90,4 | 92,7 |

**Beispiel 18: Vergleichende Hydrolyse von Napro 18/12 Fischöl**

In 5 Kolben ausgestattet mit Rührfischen wurden jeweils 50 g Wasser und 50 g Napro 18/12 Fischöl gegeben. Zu Ansatz 1 wurden 120 U Candida antarctica A Lipase gegeben. Zu Ansatz 2 wurden 120 U Candida rugosa Lipase gegeben. Zu Ansatz 3 wurde eine Mischung aus Candida antarctica A Lipase und Candida rugosa Lipase mit einer Gesamtaktivität von 120 U gegeben. Zu Ansatz 4 wurden 120 U Candida cylindracea Lipase gegeben. Zu Ansatz 5 wurde eine Mischung aus Candida antarctica A Lipase und Candida cylindracea Lipase mit einer Gesamtaktivität von 120 U gegeben. Die Ansätze wurden bei 45°C unter Rühren inkubiert. Nach 1 h, 2 h, 3 h, 5 h, 7 h, 24 h und 48 h wurden jeweils Proben aus den Ansätzen entnommen. Die Ölphase wurde von der Wasserphase separiert und die Proben wurden auf ihre Säurezahl hin untersucht. Aus der gemessenen Säurezahl wurde der entsprechende Hydrolysegrad der Fischöle berechnet auf Basis einer maximal erreichbaren Säurezahl von 195 bei vollständiger Hydrolyse.

Tabelle 16: Hydrolysegrad [in %] in Abhängigkeit der Reaktionszeit

| Zeit | Ansatz 1 | Ansatz 2 | Ansatz 3 | Ansatz 4 | Ansatz 5 |
|---|---|---|---|---|---|
| **0 h** | 0% | 0% | 0% | 0% | 0% |
| 1 h | 14,5 % | 17,9 % | 20,2 % | 28,1 % | 23 % |
| 2 h | 19 % | 23 % | 23,4 % | 32,8 % | 28,5 % |
| 3 h | 23,5 % | 26 % | 27,6 % | 37 % | 33,3 % |
| 5 h | 26,7 % | 28,3 % | 30,8 % | 38,9 % | 37,4 % |
| 7 h | 30,2 % | 30,7 % | 35,2 % | 42,2 % | 42,9 % |
| 24 h | 39,8 % | 38,7 % | 42,7 % | 48,3 % | 51,1 % |
| 48 h | 42,6 % | 40,4 % | 46,4 % | 49,8 % | 54 % |

**Fig. 1**: Die Hydrolysegrade der Ansätze 1 und 2 wurden mit dem Ansatz 3, der die Enzymmischung bei gleicher Gesamtenzymaktivität enthält, graphisch verglichen (Crug = Candida rugosa Lipase (Ansatz 2), Cant = Candida antarctica A Lipase (Ansatz 1) und Mixed = Mischung beider Lipasen (Ansatz 3)).

**Fig. 2:** Die Hydrolysegrade der Ansätze 1 und 4 wurden mit dem Ansatz 5, der die Enzymmischung bei gleicher Gesamtenzymaktivität enthält, graphisch verglichen (Ccyl = Candida cylindracea Lipase (Ansatz 4), Cant = Candida

antarctica A Lipase (Ansatz 1) und Mixed = Mischung beider Lipasen (Ansatz 5)).

**Fazit**: Die Mischung sowohl von Candida antarctica A Lipase mit Candida rugosa Lipase als auch die Mischung von Candida antarctica A Lipase mit Candida cylindracea Lipase zeigten eine höhere Hydrolysegeschwindigkeit als die jeweiligen Enzyme alleine bei gleicher Gesamtenzymaktivität im Reaktionsansatz. Die synergistische Wirkung zeigte sich bei der Kombination Candida antarctica A mit Candida rugosa über die gesamte Hydrolysedauer, während bei der Mischung mit Candida cylindracea die synergistische Wirkung erst ab einem Spaltgrad von 40 - 50 % signifikant wurde.

**Beispiel 19: Vergleichende Hydrolyse von Lamotte Typ 170 Fischöl**

In 5 Kolben ausgestattet mit Rührfischen wurden jeweils 50 g Wasser und 50 g Lamotte Typ 170 Fischöl gegeben. Zu Ansatz 1 wurden 120 U Candida antarctica A Lipase gegeben. Zu Ansatz 2 wurden 120 U Candida rugosa Lipase gegeben. Zu Ansatz 3 wurde eine Mischung aus Candida antarctica A Lipase und Candida rugosa Lipase mit einer Gesamtaktivität von 120 U gegeben. Zu Ansatz 4 wurden 120 U Candida cylindracea Lipase gegeben. Zu Ansatz 5 wurde eine Mischung aus Candida antarctica A Lipase und Candida cylindracea Lipase mit einer Gesamtaktivität von 120 U gegeben. Die Ansätze wurden bei 45 °C unter Rühren inkubiert. Nach 1 h, 2 h, 3 h, 5 h, 7 h, 24 h und 48 h wurden jeweils Proben aus den Ansätzen entnommen. Die Ölphase wurde von der Wasserphase separiert und die Proben wurden auf ihre Säurezahl hin untersucht. Aus der gemessenen Säurezahl wurde der entsprechende Hydrolysegrad der Fischöle berechnet auf Basis einer maximal erreichbaren Säurezahl von 195 bei vollständiger Hydrolyse.

**Tabelle 17: Hydrolysegrad [in %] in Abhängigkeit der Reaktionszeit**

| Zeit | Ansatz 1 | Ansatz 2 | Ansatz 3 | Ansatz 4 | Ansatz 5 |
|---|---|---|---|---|---|
| 0 h | 0% | 0% | 0% | 0% | 0% |
| 1 h | 17,3 % | 13,5 % | 18 % | 25,4 % | 16,2 % |
| 2 h | 22,9 % | 17,8 % | 25,3 % | 31 % | 22,3 % |
| 3 h | 26% | 23,5 % | 27,6% | 37 % | 33,3 % |
| 5 h | 28 % | 26,9 % | 33,5 % | 37,6 % | 36,5 % |
| 7 h | 32,9 % | 32 % | 39,6 % | 39,6 % | 41,6 % |
| 24 h | 35,6 % | 36,4 % | 41,5 % | 43,1 % | 43,6 % |
| 48 h | 38,5 % | 39,8 % | 42,7 % | 45,1 % | 46,2 % |

**Fig. 3**: Die Hydrolysegrade der Ansätze 1 und 2 wurden mit dem Ansatz 3, der die Enzymmischung bei gleicher Gesamtenzymaktivität enthält, graphisch verglichen (Crug = Candida rugosa Lipase (Ansatz 2), Cant = Candida antarctica A Lipase (Ansatz 1) und Mixed = Mischung beider Lipasen (Ansatz 3)).

**Fig. 4**: Die Hydrolysegrade der Ansätze 1 und 4 wurden mit dem Ansatz 5, der die Enzymmischung bei gleicher Gesamtenzymaktivität enthält, graphisch verglichen (Ccyl = Candida cylindracea Lipase (Ansatz 4), Cant = Candida antarctica A Lipase (Ansatz 1) und Mixed = Mischung beider Lipasen (Ansatz 5)).

**Fazit**: Die Mischung sowohl von Candida antarctica A Lipase mit Candida rugosa Lipase als auch die Mischung von Candida antarctica A Lipase mit Candida cylindracea Lipase zeigten eine höhere Hydrolysegeschwindigkeit als die jeweiligen Enzyme alleine bei gleicher Gesamtenzymaktivität im Reaktionsansatz. Die synergistische Wirkung zeigte sich bei der Kombination Candida antarctica A mit Candida rugosa über die gesamte Hydrolysedauer, während bei der Mischung mit Candida cylindracea die synergistische Wirkung erst ab einem Spaltgrad von 40 - 50 % signifikant wurde.

**Beispiel 20: Vergleichende Hydrolyse von Napro 18/12 Fischöl**

Die Ansätze 1, 2 und 3 aus Beispiel 18 wurden auf ihre Fettsäurezusammensetzung hin verglichen. Dazu wurden die Proben aus der Umsetzung nach 1 h, 2 h, 3 h, 5 h, 7 h, 24 h und 48 h syliliert und gaschromatographisch analysiert. Aus dem Spektrum der freigesetzten Fettsäuren kombiniert mit dem Hydrolysegrad bestimmt nach Säurezahl wurde die Zusammensetzung der freigesetzten Fettsäuren und rechnerisch die Zusammensetzung der am Glycerid gebundenen Fettsäuren bestimmt. Als Basis für die Berechnung diente die Ausgangszusammensetzung des Napro 18/12 Fischöls. Angegeben sind im Folgenden die summierten Fettsäureverteilungen an omega3-Fettsäuren (C18:3, C18:4, C20:5, C22:5 und C22:6), gesättigten Fettsäuren sowie an der Summe der gesättigten und ungesättigten Fettsäuren mit einer Kettenlänge von C14 - C 18.

**Tabelle 18: Auftragung der Fettsäurezusammensetzungen in der Glycerid-Fraktion und in den freigesetzten Fettsäuren in Abhängigkeit des Hydrolysegrades (Angaben jeweils in %)**

| Hydrolysegrad | Omega-3 Fettsäure | Omega-3 Glycerid | Gesättigt Fettsäure | Gesättigt Glycerid | C14-C18 Fettsäure | C14-C18 Glycerid |
|---|---|---|---|---|---|---|
| **Ansatz 1** | | | | | | |
| 0 | | 37,6 | | 29,3 | | 55 |
| 14,5 | 2 | 43,7 | 82,9 | 20,2 | 95,9 | 48 |
| 19 | 5,1 | 45,2 | 82,1 | 16,9 | 93 | 46,1 |
| 23,5 | 4,8 | 47,7 | 74 | 15,6 | 92,1 | 43,6 |
| 26,7 | 6,6 | 48,9 | 68,4 | 15,1 | 88,5 | 42,8 |
| 30,2 | 6,8 | 50,9 | 58,5 | 16,7 | 88,8 | 40,4 |
| 39,8 | 11,1 | 55,1 | 55,1 | 12,3 | 84,6 | 35,5 |
| 42,6 | 11,2 | 57,2 | 51,1 | 13,1 | 82 | 35 |
| **Ansatz 2** | | | | | | |
| 0 | | 37,6 | | 29,3 | | 55 |
| 17,9 | 4,1 | 44,9 | 35,5 | 27,9 | 94,9 | 46,3 |
| 23 | 3,5 | 47,8 | 40,8 | 25,9 | 95,9 | 42,8 |
| 26 | 5,2 | 49 | 40,9 | 25,2 | 94,2 | 41,2 |
| 28,3 | 5 | 50,4 | 43,1 | 23,6 | 93,5 | 39,8 |
| 30,7 | 6,2 | 51,5 | 42,5 | 23,4 | 92,6 | 38,4 |
| 38,7 | 7,9 | 56,3 | 44,5 | 19,7 | 89,8 | 33,1 |
| 40,4 | 9,6 | 56,6 | 44,2 | 19,2 | 89,1 | 31,9 |
| **Ansatz 3** | | | | | | |
| 0 | | 37,6 | | 29,3 | | 55 |
| 20,2 | 3,1 | 46,3 | 61,4 | 21,2 | 96,3 | 44,5 |
| 23,4 | 3,9 | 47,9 | 61,3 | 19,5 | 94,6 | 42,9 |
| 27,6 | 4,1 | 50,3 | 58,1 | 18,3 | 93,8 | 40,3 |
| 30,8 | 5,3 | 52 | 56,7 | 17,1 | 92,1 | 38,5 |
| 35,2 | 7,1 | 54,1 | 49 | 18,6 | 89,2 | 36,4 |
| 42,7 | 9,9 | 58,3 | 48,5 | 15 | 87,4 | 30,8 |
| 46,4 | 10,4 | 61,1 | 46,3 | 14,6 | 84,5 | 29,4 |

**Fig. 5:** Vergleichende Auftragung der omega 3 Fettsäuren der einzelnen Ansätze in Glycerid- und Fettsäure Fraktion (Cant Hydrolysat = freigesetzte Fettsäuren aus Ansatz 1; Cant Glycerid = Glycerid gebundene Fettsäuren aus Ansatz 1, Crug Hydrolysat = freigesetzte Fettsäuren aus Ansatz 2; Crug Glycerid = Glycerid gebundene Fettsäuren aus Ansatz 2, Mixed Hydrolysat = freigesetzte Fettsäuren aus Ansatz 3; Mixed Glycerid = Glycerid gebundene Fettsäuren aus Ansatz 3)

**Fig. 6**: Vergleichende Auftragung der gesättigten Fettsäuren der einzelnen Ansätze in Glycerid- und Fettsäure Fraktion (Cant Hydrolysat = freigesetzte Fettsäuren aus Ansatz 1; Cant Glycerid = Glycerid gebundene Fettsäuren aus Ansatz 1, Crug Hydrolysat = freigesetzte Fettsäuren aus Ansatz 2; Crug Glycerid = Glycerid gebundene Fettsäuren aus Ansatz 2, Mixed Hydrolysat = freigesetzte Fettsäuren aus Ansatz 3; Mixed Glycerid = Glycerid gebundene Fettsäuren aus Ansatz 3)

**Fig. 7**: Vergleichende Auftragung der gesättigten und ungesättigten Fettsäuren einer Kettenlänge von C14 - C 18 der einzelnen Ansätze in Glycerid- und Fettsäure Fraktion (Cant Hydrolysat = freigesetzte Fettsäuren aus Ansatz 1; Cant Glycerid = Glycerid gebundene Fettsäuren aus Ansatz 1, Crug Hydrolysat = freigesetzte Fettsäuren aus Ansatz 2; Crug Glycerid = Glycerid gebundene Fettsäuren aus Ansatz 2, Mixed Hydrolysat = freigesetzte Fettsäuren aus Ansatz 3; Mixed Glycerid = Glycerid gebundene Fettsäuren aus Ansatz 3)

**Fazit**: Die Abspaltung von C14 - C 18 Fettsäuren aus dem Glycerid insgesamt war bei beiden eingesetzten Enzymen vergleichbar, auch die negative Selektivität gegenüber omega-3 Fettsäuren mit einem Hauptanteil an Eicosapentaen- und Docosahexaensäure. Die Mischung der beiden Enzyme verhielt sich hier vergleichbar zu den Einzelenzymen. Ein deutlicher Unterschied war sichtbar bei der Abspaltung der gesättigten Fettsäuren aus dem Glycerid. Hier war eine deutliche Präferenz von Candida antarctica A Lipase zu erkennen. Entsprechend bevorzugte die Candida rugosa Lipase eher die ungesättigten C14 - C18 Fettsäuren, wodurch der ähnliche Verlauf der C14-C18 Fettsäuren

gesamt zu erklären ist. Dieser Unterschied bewirkte die synergistische Wirkung der beiden Enzyme in Bezug auf die erhöhte Hydrolysegeschwindigkeit wie gezeigt in Beispiel 5.

**Beispiel 21: Vergleichende Hydrolyse von Napro 18/12 Fischöl**

Die Ansätze 1, 4 und 5 aus Beispiel 18 wurden auf ihre Fettsäurezusammensetzung hin verglichen. Dazu wurden die Proben aus der Umsetzung nach 1 h, 2 h, 3 h, 5 h, 7 h, 24 h und 48 h syliliert und gaschromatographisch analysiert. Aus dem Spektrum der freigesetzten Fettsäuren kombiniert mit dem Hydrolysegrad bestimmt nach Säurezahl wurde die Zusammensetzung der freigesetzten Fettsäuren und rechnerisch die Zusammensetzung der am Glycerid gebundenen Fettsäuren bestimmt. Als Basis für die Berechnung diente die Ausgangszusammensetzung des Napro 18/12 Fischöls. Angegeben sind im Folgenden die summierten Fettsäureverteilungen an omega3-Fettsäuren (C18:3, C18:4, C20:5, C22:5 und C22:6), gesättigten Fettsäuren sowie an der Summe der gesättigten und ungesättigten Fettsäuren mit einer Kettenlänge von C14 - C 18.

**Tabelle 19: Auftragung der Fettsäurezusammensetzungen in der Glycerid-Fraktion und in den freigesetzten Fettsäuren in Abhängigkeit des Hydrolysegrades (Angaben jeweils in %)**

| **Hydrolysegrad** | **omega 3** Fettsäure | **omega 3** Glycerid | **Gesättigt** Fettsäure | **Gesättigt** Glycerid | **C14- C18** Fettsäure | **C14- C18** Glycerid |
|---|---|---|---|---|---|---|
| **Ansatz 1** | | | | | | |
| 0 | | 37,6 | | 29,3 | | 55 |
| 14,5 | 2 | 43,7 | 82,9 | 20,2 | 95,9 | 48 |
| 19 | 5,1 | 45,2 | 82,1 | 16,9 | 93 | 46,1 |
| 23,5 | 4,8 | 47,7 | 74 | 15,6 | 92,1 | 43,6 |
| 26,7 | 6,6 | 48,9 | 68,4 | 15,1 | 88,5 | 42,8 |
| 30,2 | 6,8 | 50,9 | 58,5 | 16,7 | 88,8 | 40,4 |
| 39,8 | 11,1 | 55,1 | 55,1 | 12,3 | 84,6 | 35,5 |
| 42,6 | 11,2 | 57,2 | 51,1 | 13,1 | 82 | 35 |
| **Ansatz 4** | | | | | | |
| 0 | | 37,6 | | 29,3 | | 55 |
| 28,1 | 6,4 | 49,8 | 39,8 | 25,2 | 92,6 | 40,3 |
| 32,8 | 9,7 | 51,2 | 37,8 | 25,1 | 87,3 | 39,2 |
| 37 | 9,6 | 54 | 39,2 | 23,5 | 86,3 | 36,7 |
| 38,9 | 11,2 | 54,4 | 38,2 | 23,6 | 84,7 | 36 |
| 42,2 | 11,6 | 56,6 | 35,6 | 24,7 | 85,2 | 33 |
| 48,3 | 14,1 | 59,6 | 39,3 | 20 | 81,5 | 30,3 |
| 49,8 | 13,2 | 61,9 | 40,4 | 18,2 | 82,9 | 27,3 |
| **Ansatz 5** | | | | | | |
| 0 | | 37,6 | | 29,3 | | 55 |
| 23 | 5 | 47,3 | 55,4 | 21,5 | 92,9 | 43,7 |
| 28,5 | 4,6 | 50,7 | 55 | 19,1 | 94,1 | 39,5 |
| 33,3 | 6,8 | 53 | 50,9 | 18,5 | 90 | 37,5 |
| 37,4 | 6,9 | 56 | 51,1 | 16,2 | 90,3 | 33,9 |
| 42,9 | 10,7 | 57,8 | 46 | 16,7 | 85 | 32,5 |
| 51,1 | 11,6 | 64,9 | 44,5 | 13,4 | 84,2 | 24,5 |
| 54 | 14,3 | 65 | 40,9 | 15,7 | 80,3 | 25,2 |

**Fig. 8:** Vergleichende Auftragung der omega 3 Fettsäuren der einzelnen Ansätze in Glycerid- und Fettsäure Fraktion (Cant Hydrolysat = freigesetzte Fettsäuren aus Ansatz 1; Cant Glycerid = Glycerid gebundene Fettsäuren aus Ansatz 1, Ccyl Hydrolysat = freigesetzte Fettsäuren aus Ansatz 4; Ccyl Glycerid = Glycerid gebundene Fettsäuren aus Ansatz 4, Mixed Hydrolysat = freigesetzte Fettsäuren aus Ansatz 5; Mixed Glycerid = Glycerid gebundene Fettsäuren aus Ansatz 5)

**Fig. 9**: Vergleichende Auftragung der gesättigten Fettsäuren der einzelnen Ansätze in Glycerid- und Fettsäure Fraktion (Cant Hydrolysat = freigesetzte Fettsäuren aus Ansatz 1; Cant Glycerid = Glycerid gebundene Fettsäuren aus Ansatz 1, Ccyl Hydrolysat = freigesetzte Fettsäuren aus Ansatz 4; Ccyl Glycerid = Glycerid gebundene Fettsäuren aus

Ansatz 4, Mixed Hydrolysat = freigesetzte Fettsäuren aus Ansatz 5; Mixed Glycerid = Glycerid gebundene Fettsäuren aus Ansatz 5)

**Fig. 10**: Vergleichende Auftragung der gesättigten und ungesättigten Fettsäuren einer Kettenlänge von C14 - C 18 der einzelnen Ansätze in Glycerid- und Fettsäure Fraktion (Cant Hydrolysat = freigesetzte Fettsäuren aus Ansatz 1; Cant Glycerid = Glycerid gebundene Fettsäuren aus Ansatz 1, Ccyl Hydrolysat = freigesetzte Fettsäuren aus Ansatz 4; Ccyl Glycerid = Glycerid gebundene Fettsäuren aus Ansatz 4, Mixed Hydrolysat = freigesetzte Fettsäuren aus Ansatz 5; Mixed Glycerid = Glycerid gebundene Fettsäuren aus Ansatz 5)

**Fazit**: Die Abspaltung von C14 - C 18 Fettsäuren aus dem Glycerid insgesamt war bei beiden eingesetzten Enzymen vergleichbar, auch die negative Selektivität gegenüber omega 3 Fettsäuren mit einem Hauptanteil an Eicosapentaen- und Docosahexaensäure. Die Mischung der beiden Enzyme verhielt sich hier vergleichbar zu den Einzelenzymen. Ein deutlicher Unterschied war sichtbar bei der Abspaltung der gesättigten Fettsäuren aus dem Glycerid. Hier war eine deutliche Präferenz von Candida antarctica A Lipase zu erkennen. Entsprechend bevorzugte die Candida cylindracea Lipase eher die ungesättigten C14 - C 18 Fettsäuren, wodurch der ähnliche Verlauf der C14-C18 Fettsäuren gesamt zu erklären ist. Dieser Unterschied bewirkte die synergistische Wirkung der beiden Enzyme in Bezug auf die erhöhte Hydrolysegeschwindigkeit wie gezeigt in Beispiel 18.

**Beispiel 22: Vergleichende Hydrolyse von Lamotte Typ 170 Fischöl**

Die Ansätze 1, 2, 3, 4 und 5 aus Beispiel 19 wurden auf ihre Fettsäurezusammensetzung hin verglichen. Dazu wurden die Proben aus der Umsetzung nach 1 h, 2 h, 3 h, 5 h, 7 h, 24 h und 48 h syliliert und gaschromatographisch analysiert. Aus dem Spektrum der freigesetzten Fettsäuren kombiniert mit dem Hydrolysegrad bestimmt nach Säurezahl wurde die Zusammensetzung der freigesetzten Fettsäuren und rechnerisch die Zusammensetzung der am Glycerid gebundenen Fettsäuren bestimmt. Als Basis für die Berechnung diente die Ausgangszusammensetzung des Napro 18/12 Fischöls. Angegeben sind im Folgenden die summierten Fettsäureverteilungen an omega3-Fettsäuren (C18:3, C18:4, C20:5, C22:5 und C22:6), gesättigten Fettsäuren sowie an der Summe der gesättigten und ungesättigten Fettsäuren mit einer Kettenlänge von C14-C18.

**Fazit:** Die Abspaltung von C14-C18 Fettsäuren aus dem Glycerid insgesamt war bei allen eingesetzten Enzymen vergleichbar, auch die negative Selektivität gegenüber omega 3 Fettsäuren mit einem Hauptanteil an Eicosapentaen- und Docosahexaensäure. Die jeweilige Mischung der beiden Enzyme verhielt sich hier vergleichbar zu den Einzelenzymen. Ein deutlicher Unterschied war sichtbar bei der Abspaltung der gesättigten Fettsäuren aus dem Glycerid. Hier war eine deutliche Präferenz von Candida antarctica A Lipase zu erkennen. Entsprechend bevorzugte die Candida rugosa Lipase und auch die Candida cylindracea Lipase eher die ungesättigten C14 - C18 Fettsäuren, wodurch der ähnliche Verlauf der C14-C18 Fettsäuren gesamt zu erklären ist. Dieser Unterschied bewirkte die synergistische Wirkung der beiden Enzyme in Bezug auf die erhöhte Hydrolysegeschwindigkeit wie gezeigt in Beispiel 19.

**Beispiel 23: Selektive Umesterung von Napro 11/19 Fischöl mit Candida antarctica A**

A) Enzymimmobilisation

80 g Lewatit VPOC 1600 (Lanxess) und 1500 ml Wasser wurden in einen 2,5 1 Reaktor gegeben. Unter Rühren wurden 120 g einer kommerziell erhältlichen Lipase A aus Candida antarctica (Novozym® 735) gegeben. Das Gemisch wurde für 3 h inkubiert und anschließend abfiltriert. Das Immobilisat wurde feucht für die Umesterung eingesetzt.

B) Reaktion + Aufarbeitung

1000 g Fischöl 11/19 (ein Fischöl der Zusammensetzung wie angegeben in der folgenden Tabelle) 100 g Wasser und 100 g Ethanol wurden in einen Doppelmantelreaktor gegeben und unter Rühren auf 45 °C aufgeheizt. Zum Ansatz wurde das feuchte Enzymimmobilisat gegeben und der Ansatz wurde bei konstanter Temperatur unter Rühren 24 h inkubiert. Die Säurezahl im Produktgemisch betrug 15,6. Nach Phasenseparation wurde die wässrige Phase abgetrennt und die Ölphase bei 80 °C im Rotationsverdampfer getrocknet. Die Ölphase wurde an einer Kurzwegdestillation aufgearbeitet. Bei einer Temperatur von 190°C und einem Vakuum von 0,2 mbar wurden gebildete Ethylester und freie Säuren über Kopf destilliert. Das Sumpfprodukt ist das angereicherte PUFA-Glycerid. Die Massenbilanz von Destillat und Sumpfprodukt ergab einen Umsetzungsgrad des Öls von 50 %, wobei knapp 20 % als freie Säuren und über 80 % der abgespaltenen Fettsäuren in Form der Ethylester vorlagen.

C) Produktanalyse

Das Produkt und vergleichend das Ausgangsmaterial wurde mit GPC (Gelpermeationschromatographie)

(Glyceridverteilung); GC (Gaschromatographie) (Fettsäuremuster nach Methylierung) analysiert, sowie die Säurezahl gemessen. Die Ergebnisse sind in Tabelle 20 zusammengefasst.

**Tabelle 20: Fettsäureverteilung und Zusammensetzung im Glycerid und im Ausgangsprodukt**

| Fettsäure | Fischöl 11/19 [%] | PUFA-Glycerid [%] | Ab-/Anreicherung |
|---|---|---|---|
| 14:0 | 5,0 | 1,3 | |
| 16:0 | 15,3 | 3,8 | |
| 16:1 | 5,8 | 5,1 | |
| 16:4 | 1,0 | 1,4 | |
| 18:0 | 3,6 | 1,4 | |
| 18:1 | 8,3 | 7,4 | |
| 18:4 | 3,9 | 6,2 | |
| 20:4 | 0,9 | 1,4 | |
| 20:5 | 14,0 | 22,5 | |
| 22:1 | 2,2 | 1,7 | |
| 22:5 | 1,8 | 3 | |
| 22:6 | 19,6 | 33,7 | |
| | | | |
| Summe SAFA (14,16,18) | 23,9 | 6,5 | 3,7 |
| Summe MUFA | 16,3 | 14,2 | 1,1 |
| Summe PUFA | 41,2 | 68,2 | 1,7 |
| Summe omega3 | 39,3 | 65,4 | 1,7 |
| Summe 5 und 6 fach ungesättigte Fettsäuren | 35,4 | 59,2 | 1,7 |
| Zugeordnet | 81,4 | 88,9 | |
| | | | |
| Fettsäure + Ethylester | | 1,5 | |
| Monoglycerid | | 6,1 | |
| Diglycerid | | 43,5 | |
| Triglycerid | | 48 | |
| Säurezahl | | 2,1 | |

**Beispiel 24: Selektive Umesterung von Napro 11/19 Fischöl mit Candida antarctica A**

Analog zu Beispiel 23 wurde eine Reaktion durchgeführt, die 48 h inkubiert wurde. Die Säurezahl im Produktgemisch betrug 27. Die Massenbilanz von Destillat und Sumpfprodukt ergab einen Umsetzungsgrad des Öls von 65 %, wobei etwa 20 % als freie Säuren und etwa 80 % der abgespaltenen Fettsäuren in Form der Ethylester vorlagen. Die Ergebnisse sind in Tabelle 16 zusammengefasst. Die Destillation wurde bei einem Vakuum von 0,06 mbar und einer Temperatur von 180 °C durchgeführt.

**Tabelle 21: Fettsäureverteilung und Zusammensetzung im Glycerid und im Ausgangsprodukt**

| Fettsäure | Fischöl 11/19 [%] | PUFA-Glycerid [%] | Ab-/Anreicherung |
|---|---|---|---|
| 14:0 | 5,0 | 1,6 | |
| 16:0 | 15,3 | 4,7 | |
| 16:1 | 5,8 | 2,8 | |
| 16:4 | 1,0 | 1,6 | |
| 18:0 | 3,6 | 1,2 | |
| 18:1 | 8,3 | 4,9 | |
| 18:4 | 3,9 | 6,3 | |
| 20:4 | 0,9 | 1,4 | |
| 20:5 | 14,0 | 22,2 | |
| 22:5 | 1,8 | 2,9 | |
| 22:6 | 19,6 | 39,3 | |

(fortgesetzt)

| Fettsäure | Fischöl 11/19 [%] | PUFA-Glycerid [%] | Ab-/Anreicherung |
|---|---|---|---|
| Summe SAFA (14,16,18) | 23,9 | 7,5 | 3,2 |
| Summe MUFA | 14,1 | 7,7 | 1,8 |
| Summe PUFA | 41,2 | 73,7 | 1,8 |
| Summe omega3 | 39,3 | 70,7 | 1,8 |
| Summe 5 und 6 fach. ungesättigte Fettsäuren | 35,4 | 64,4 | 1,8 |
| Zugeordnet | 79,2 | 88,9 | |
| | | | |
| Fettsäure + Ethylester | | 0,8 | |
| Monoglycerid | | 2,6 | |
| Diglycerid | | 44,9 | |
| Triglycerid | | 51,7 | |

**Beispiel 25: Selektive Umesterung von Napro 18/12 Fischöl mit Candida antarctica A**

Analog zu Beispiel 23 wurde eine Reaktion mit Napro 18/12 Fischöl durchgeführt. Die Säurezahl im Produktgemisch betrug 24. Die Massenbilanz von Destillat und Sumpfprodukt ergab einen Umsetzungsgrad des Öls von 60 %, wobei etwa 20 % als freie Säuren und etwa 80 % der abgespaltenen Fettsäuren in Form der Ethylester vorlagen. Die Ergebnisse sind in Tabelle 17 zusammengefasst. Die Destillation wurde bei einem Vakuum von 0,06 mbar und einer Temperatur von 180 °C durchgeführt.

**Tabelle 22: Fettsäureverteilung und Zusammensetzung im Glycerid und im Ausgangsprodukt**

| Fettsäure | Fischöl 11/19 [%] | PUFA-Glycerid [%] | Ab-/Anreicherung |
|---|---|---|---|
| Fettsäure | Napro18/12 | Muster ex 18/12 | |
| 14:0 | 6,7 | 1,6 | |
| 16:0 | 16,6 | 4,2 | |
| 16:1 | 8 | 4,5 | |
| 16:4 | 2,1 | 3,1 | |
| 18:0 | 3,4 | 0,9 | |
| 18:1 | 12,2 | 6,5 | |
| 18:2 | 1,3 | 0,8 | |
| 18:3 | 0,9 | 0,6 | |
| 18:4 | 3,3 | 5,5 | |
| 20:1 | 1,5 | 0,7 | |
| 20:4 | 1,1 | 1,5 | |
| 20:5 | 18,4 | 30 | |
| 22:5 | 2,2 | 3,4 | |
| 22:6 | 12,8 | 27,5 | |
| | | | |
| Summe SAFA (14,16,18) | 26,7 | 6,7 | 4 |
| Summe MUFA | 21,7 | 11,7 | 1,9 |
| Summe PUFA | 40,8 | 71,6 | 1,8 |
| Summe omega3 | 37,6 | 67 | 1,8 |
| Summe 5 und 6 fach ungesättigte Fettsäuren | 33,4 | 60,9 | 1,8 |
| Zugeordnet | 90,5 | 90,8 | |
| | | | |
| Fettsäure + Ethylester | | 0,7 | |
| Monoglycerid | | 1,9 | |

(fortgesetzt)

| Fettsäure | Fischöl 11/19 [%] | PUFA-Glycerid [%] | Ab-/Anreicherung |
|---|---|---|---|
| Diglycerid | | 45,4 | |
| Triglycerid | | 52,1 | |

Im Folgenden werden die Fig. 11 bis 14 beschrieben.

Fig 11: Grafische Darstellung der maximalen und halbmaximalen Gehalte an fünffach und sechsfach ungesättigten Fettsäuren sowie die entsprechenden Gehalte an gesättigten Fettsäuren, die in der Glyceridfraktion gebunden sind, für einen Umsetzungsgrad von 0 - 70 % berechnet für das Fischöl Napro 18/12.

Fig 12: Grafische Darstellung der maximalen und halbmaximalen Gehalte an fünffach und sechsfach ungesättigten Fettsäuren sowie die entsprechenden Gehalte an gesättigten Fettsäuren, die in der Glyceridfraktion gebunden sind, für einen Umsetzungsgrad von 0 - 70 % berechnet für das Fischöl Napro 11/19.

Fig. 13: Grafische Darstellung der maximalen und halbmaximalen Gehalte an fünffach und sechsfach ungesättigten Fettsäuren sowie die entsprechenden Gehalte an gesättigten Fettsäuren, die in der Glyceridfraktion gebunden sind, für einen Umsetzungsgrad von 0 - 70 % berechnet für das Fischöl Lamotte Typ 170.

Fig 14: Grafische Darstellung der maximalen und halbmaximalen Gehalte an fünffach und sechsfach ungesättigten Fettsäuren sowie die entsprechenden Gehalte an gesättigten Fettsäuren, die in der Glyceridfraktion gebunden sind, für einen Umsetzungsgrad von 0 - 70 % berechnet für das Fischöl Brudy Algatrium DHA 18.

In Fig. 11 bis 14 werden folgende Symbole verwendet:

▲ = Maximaler Gehalt an fünf- und sechsfach ungesättigten Fettsäuren in % in Abhängigkeit des Umsetzungsgrades
Durchgezogene Linie = Gehalt an fünf- und sechsfach ungesättigten Fettsäuren in % bei nicht selektiver Umsetzung
× = Halbmaximale Anreicherung an fünf- und sechsfach ungesättigten Fettsäuren in %
■ = Anteil der gesättigten Fettsäuren bei maximaler Anreicherung an fünf- und sechsfach ungesättigten Fettsäuren in %
Gestrichelte Linie = Gehalt an gesättigten Fettsäuren in % bei nicht selektiver Umsetzung
● = Anteil der gesättigten Fettsäuren bei halbmaximaler Anreicherung an fünf- und sechsfach ungesättigten Fettsäuren in %

## Patentansprüche

1. Ein Gemisch enthaltend

(A) optional mindestens ein Monoglycerid der Formel (I) oder (II),

(I)

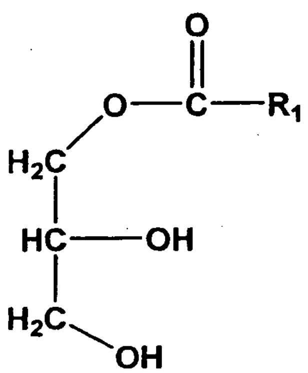

(II)

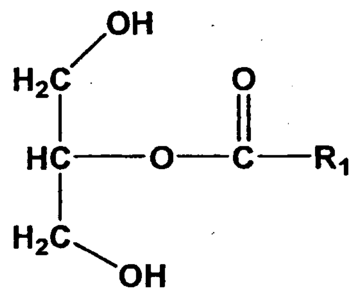

(B) mindestens ein Diglycerid der Formel (III) oder (IV) und

(III)

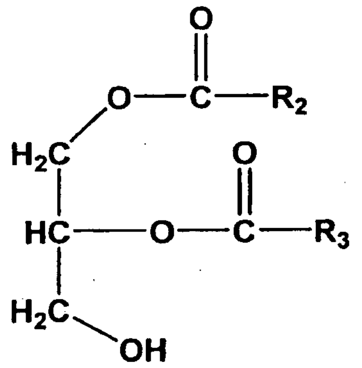

(IV)

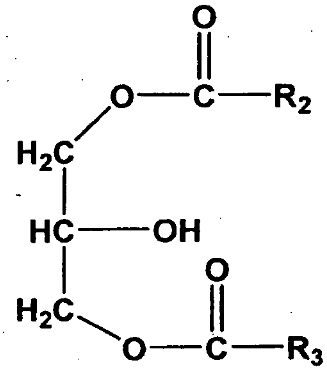

(D) mindestens ein Triglycerid der Formel (V),

(V)

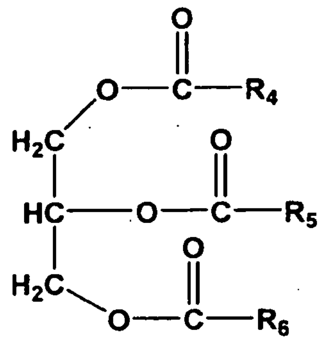

wobei die Reste $R_1$-CO-, $R_2$-CO-, $R_3$-CO-, $R_4$-CO-, $R_5$-CO- und $R_6$-CO- unabhängig voneinander ausgewählt werden aus der Gruppe bestehend aus einem gesättigten Fettsäureacylrest, einem einfach bis vierfach ungesättigten Fettsäureacylrest und einem mindestens fünffach ungesättigten Fettsäureacylrest,

und wobei die Summe der Massen aller in dem Gemisch vorhandenen mindestens fünffach ungesättigten Fettsäureacylreste, berechnet als freie Fettsäuren, bezogen auf die Summe der Massen aller in dem Gemisch vorhandenen Fettsäureacylreste, wiederum berechnet als freie Fettsäuren, mindestens 40 Gew.-% und höchstens 80 Gew.-% beträgt,
und wobei die Summe der Massen aller in dem Gemisch vorhandenen gesättigten Fettsäureacylreste, berechnet als freie Fettsäure, bezogen auf die Summe der Massen aller in dem Gemisch vorhandenen Fettsäureacylreste, wiederum berechnet als freie Fettsäuren, höchstens

$$\frac{85-a}{3} \text{ Gew.-\%}$$

beträgt, wobei *a* die Summe der Massen aller in dem Gemisch vorhandenen mindestens fünffach ungesättigten Fettsäureacylreste, berechnet als freie Fettsäuren, bezogen auf die Summe der Massen aller in dem Gemisch

vorhandenen Fettsäureacylreste, wiederum berechnet als freie Fettsäuren, in Gew.-% ist,
und wobei die Masse an Fettsäureacylresten der Stearinsäure, berechnet als freie Fettsäure, bezogen auf die Summe der Massen aller in dem Gemisch vorhandenen Fettsäureacylreste, wiederum berechnet als freie Fettsäuren, höchstens 2 Gew.-% beträgt,
und wobei der Gehalt an Triglyceriden bezogen auf alle Glyceride der Formeln I - V 50 bis 85 Gew.-% beträgt und zusätzlich die Bedingung erfüllt, dass der Gehalt an Triglyceriden bezogen auf alle Glyceride der Formeln I - V mindestens

$$\frac{410 - 5 \cdot a}{3} \text{ Gew.-\%}$$

beträgt, wobei a die oben definierte Bedeutung hat,
und wobei, der Gehalt an Diglyceriden bezogen auf alle Glyceride der Formeln I - V mindestens

$$100 - 3 - b \text{ Gew.-\%}$$

beträgt, wobei b der Gehalt an Triglyceriden bezogen auf alle Glyceride der Formeln I - V in Gew.-% ist.

**2.** Die Verwendung des Gemisches nach Anspruch 1 als Nahrungsergänzungsmittel oder als Nahrungsmittelzusatzstoff für die menschliche Ernährung oder als Futtermittel für die Tierernährung.

**3.** Ein Verfahren zur Herstellung des Gemisches nach Anspruch 1 aus einem ersten Gemisch enthaltend

(A) optional mindestens ein Monoglycerid der Formel (I) oder (II),

(I)

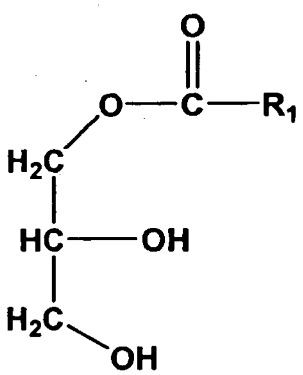

(II)

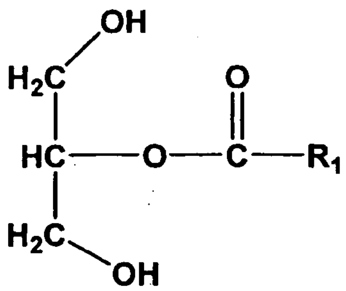

(B) optional mindestens ein Diglycerid der Formel (III) oder (IV) und

(III)

(IV)

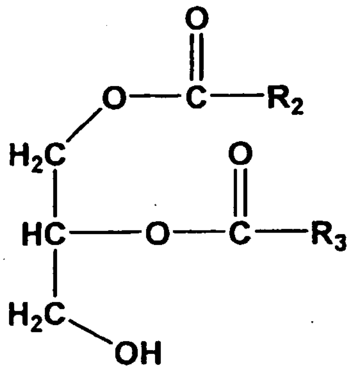

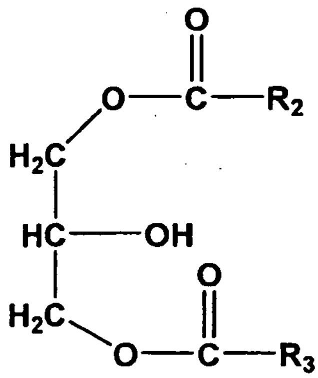

(D) mindestens ein Triglycerid der Formel (V),

(V)

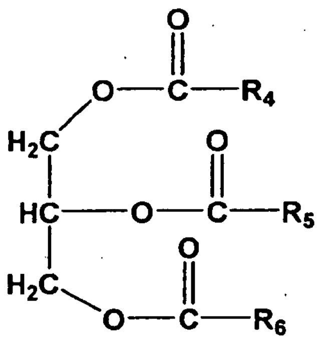

wobei die Reste $R_1$-CO-, $R_2$-CO-, $R_3$-CO-, $R_4$-CO-, $R_5$-CO- und $R_6$-CO- unabhängig voneinander ausgewählt werden aus der Gruppe bestehend aus einem gesättigten Fettsäureacylrest, einem einfach bis vierfach ungesättigten Fettsäureacylrest und einem mindestens fünffach ungesättigten Fettsäureacylrest,
und wobei die Summe der Massen aller in dem Gemisch vorhandenen mindestens fünffach ungesättigten Fettsäureacylreste, berechnet als freie Fettsäuren, bezogen auf die Summe der Massen aller in dem Gemisch vorhandenen Fettsäureacylreste, wiederum berechnet als freie Fettsäuren, mindestens um den Faktor 1,3 höher ist als im ersten Gemisch (Faktor A),
und wobei die Summe der Massen aller in dem Gemisch vorhandenen gesättigten Fettsäureacylreste, berechnet als freie Fettsäure, bezogen auf die Summe der Massen aller in dem Gemisch vorhandenen Fettsäureacylreste, wiederum berechnet als freie Fettsäuren mindestens um den Faktor 2 niedriger ist als im ersten Gemisch (Faktor B),
und wobei der Wert des Faktors A kleiner ist als der Wert des Faktors B,
und wobei der Gehalt des ersten Gemisches an Triglyceriden bezogen auf alle Glyceride der Formeln I - V mindestens 90 Gew.- % beträgt,
und wobei das erste Gemisch mindestens fünffach ungesättigten Fettsäureacylreste enthält, und wobei in einer bevorzugten Ausführungsform der vorliegenden Erfindung die Summe der Massen aller in dem ersten Gemisch vorhandenen mindestens fünffach ungesättigten Fettsäureacylreste, berechnet als freie Fettsäuren, bezogen auf die Summe der Massen aller in dem ersten Gemisch vorhandenen Fettsämeacylreste, wiederum berechnet als freie Fettsäuren, mindestens 15 Gew.-% beträgt,
und wobei das Verfahren die Umsetzung des ersten Gemisches mit Wasser oder mit einem Alkohol mit 1 bis 4 C-Atomen in Gegenwart von
Lipase A aus Candida antarctica umfasst.

**4.** Das Verfahren nach Anspruch 3, wobei die Umsetzung des ersten Gemisches mit Wasser oder mit einem Alkohol mit 1 bis 4 C-Atomen in Gegenwart einer ersten, nicht regioselektiven Lipase mit einer erhöhten Spezifität für

gesättigte Fettsäuren und einer zweiten, nicht regioselektiven Lipase mit einer erhöhten Spezifität für einfach ungesättigten Fettsäuren im Vergleich zu allen ungesättigten Fettsäuren erfolgt, wobei die erste Lipase Lipase A aus Candida antarctica ist, und wobei die zweite Lipase ausgewählt wird aus einer der Lipasen aus Candida rugosa oder Candida cylindracea.

**5.** Das Verfahren nach einem der Ansprüche 3 bis 4, wobei das erste Gemisch ausgewählt wird aus der Gruppe bestehend aus einem Fischöl, einem Öl von marinen Crustaceen, einem Öl von Mikroalgen, einem Öl von marinen Mikroorganismen und einem Öl marin lebender Säugetiere, wobei bevorzugt ein Öl mit einem Gehalt von mehr als 20 Gew.-% gebundenen mindestens fünffach ungesättigten Fettsäuren eingesetzt wird.

**6.** Das Verfahren nach einem der Ansprüche 3 bis 5, wobei die erste Lipase in freier oder immobilisierter Form eingesetzt wird.

**7.** Das Verfahren nach einem der Ansprüche 4 bis 6, wobei die zweite Lipase in freier oder immobilisierter Form eingesetzt wird.

**8.** Das Verfahren nach einem der Ansprüche 3 bis 7, wobei Wasser in einer Konzentration von 2 - 50 Gew.-% zur partiellen Hydrolyse zugegeben wird oder wobei Ethanol in einer Konzentration von 5 - 50 Gew.-% zugegeben wird, oder wobei eine Mischung von Wasser und Akohol mit einer Gesamtkonzentration von 5 - 50 % bezogen auf den Ölanteil zugegeben wird.

**Claims**

**1.** A mixture containing

(A) optionally at least one monoglyceride of the formula (I) or (II),

(I)

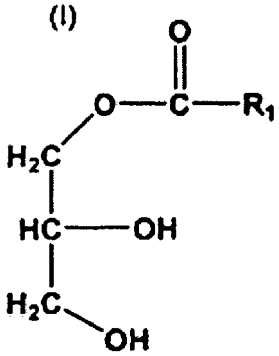

(II)

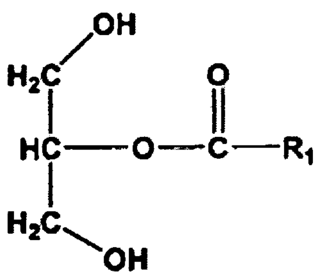

(B) at least one diglyceride of the formula (III) or (IV) and

(III)

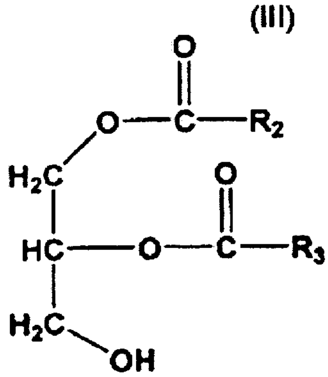

(IV)

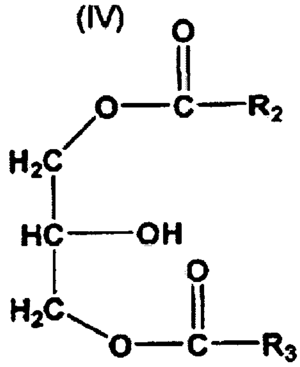

(D) at least one triglyceride of the formula (V),

(V)

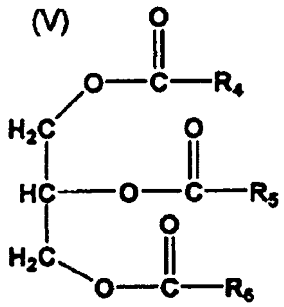

the groups $R_1$-CO-, $R_2$-CO-, $R_3$-CO-, $R_4$-CO-, $R_5$-CO- and $R_6$-CO- independently of one another being selected from the group consisting of a saturated fatty acid acyl group, a 1x- to 4x-unsaturated fatty acid acyl group and an at least 5x-unsaturated fatty acid acyl group,
and the sum of the weights of all at least 5x-unsaturated fatty acid acyl groups present in the mixture, expressed as free fatty acids, based on the sum of the weights of all fatty acid acyl groups present in the mixture, again expressed as free fatty acids, amounting to at least 40% by weight and at most 80% by weight,
and the sum of the weights of all saturated fatty acid acyl groups present in the mixture, expressed as free fatty acid, based on the sum of the weights of all fatty acid acyl groups present in the mixture, again expressed as free fatty acids, amounting to at most

$$\frac{85-a}{3}\% \text{ by weight,}$$

*a* being the sum of the weights of all at least 5x-unsaturated fatty acid acyl groups present in the mixture, expressed as free fatty acids, based on the sum of the weights of all fatty acid acyl groups present in the mixture, again expressed as free fatty acids, in % by weight,
and the weight of fatty acid acyl groups in stearic acid, expressed as free fatty acid, based on the sum of the weights of all fatty acid acyl groups present in the mixture, again expressed as free fatty acids, amounting to at most 2% by weight,
and the content of triglycerides, based on all the glycerides of the formulae I to V, being from 50 to 85% by weight and additionally meeting the requirement that the content of triglycerides, based on all the glycerides of the formulae I to V, amounts to at least

$$\frac{410 - 5\cdot a}{3}\ \%\ \text{by weight,}$$

where a is as defined above,
and the content of diglycerides, based on all the glycerides of the formulae I to V, being at least

$$100 - 3 - b\ \%\ \text{by weight,}$$

where *b* is the content of triglycerides, based on all the glycerides of the formulae I to V, in % by weight.

**2.** The use of the mixture as claimed in claim 1 as a food supplement or as a food additive for human nutrition or as a feed for animal nutrition.

**3.** A process for the production of the mixture as claimed in claim 1 from a first mixture containing

(A) optionally at least one monoglyceride of the formula (I) or (II),

(I)

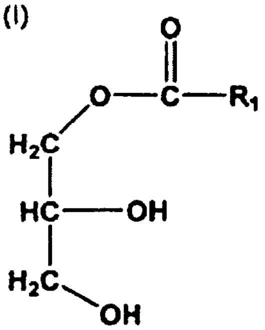

(II)

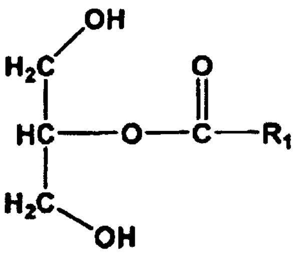

(B) optionally at least one diglyceride of the formula (III) or (IV) and

(III)

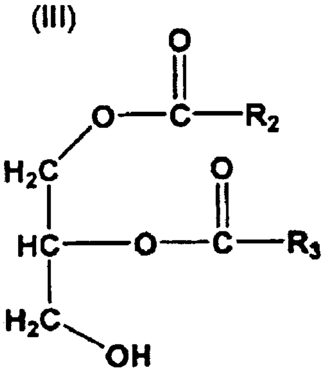

(IV)

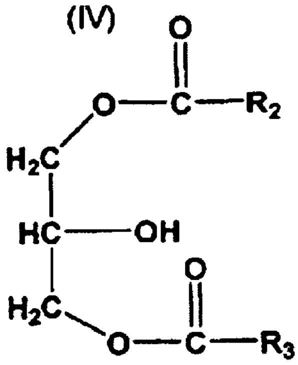

(D) at least one triglyceride of the formula (V),

(V)

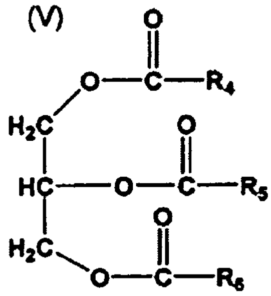

the groups $R_1$-CO-, $R_2$-CO-, $R_3$-CO-, $R_4$-CO-, $R_5$-CO- and $R_6$-CO- independently of one another being selected from the group consisting of a saturated fatty acid acyl group, a 1x- to 4x-unsaturated fatty acid acyl group and an at least 5x-unsaturated fatty acid acyl group,
and the sum of the weights of all at least 5x-unsaturated fatty acid acyl groups present in the mixture, expressed as free fatty acids, based on the sum of the weights of all fatty acid acyl groups present in the mixture, again calculated as free fatty acids, being higher by a factor of at least 1.3 than in the first mixture (factor A),
and the sum of the weights of all saturated fatty acid acyl groups present in the mixture, expressed as free fatty acid, based on the sum of the weights of all fatty acid acyl groups present in the mixture, again calculated as free fatty acids, being lower by a factor of at least 2 than in the first mixture (factor B),
and the value of factor A being smaller than the value of factor B,
and the content of triglycerides in the first mixture, based on all the glycerides of the formulae I to V, being at least 90% by weight,
and the first mixture containing at least 5x-unsaturated fatty acid acyl groups and the sum of the weights of all at least 5x-unsaturated fatty acid acyl groups present in the first mixture, expressed as free fatty acids, based on the sum of the weights of all fatty acid acyl groups present in the first mixture, again expressed as free fatty acids, in a preferred embodiment of the invention being at least 15% by weight,
and the process comprising reacting the first mixture with water or with a $C_{1-4}$ alcohol in the presence of lipase A from *Candida antarctica.*

**4.** The process as claimed in claim 3, wherein the reaction of the first mixture with water or with a $C_{1-4}$ alcohol is carried out in the presence of a first, non-regioselective lipase with an increased specificity for saturated fatty acids and a second, non-regioselective lipase with an increased specificity for monounsaturated fatty acids by comparison with all unsaturated fatty acids, wherein the first lipase is lipase A from *Candida antarctica,* and wherein the second lipase is selected from one of the lipases from *Candida rugosa* or *Candida cylindracea.*

**5.** The process as claimed in either of claims 3 or 4, wherein the first mixture is selected from the group consisting of a fish oil, an oil from marine crustaceans, an oil from microalgae, an oil from marine microorganisms and an oil from marine mammals, an oil containing more than 20% by weight bound at least 5x-unsaturated fatty acids preferably being used.

**6.** The process as claimed in any of claims 3 to 5, wherein the first lipase is used in free or immobilized form.

**7.** The process as claimed in any of claims 4 to 6, wherein the second lipase is used in free or immobilized form.

**8.** The process as claimed in any of claims 3 to 7, wherein water is added in a concentration of 2 to 50% by weight for partial hydrolysis or wherein ethanol is added in a concentration of 5 to 50% by weight or wherein a mixture of water and alcohol with a total concentration of 5 to 50%, based on the oil component, is added.

## Revendications

**1.** Mélange contenant

(A) éventuellement au moins un monoglycéride de formule (I) ou (II)

(I)

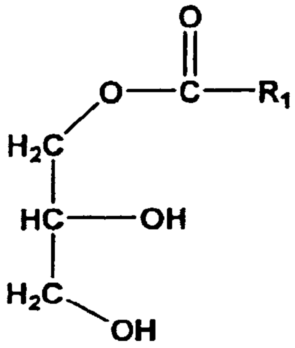

(II)

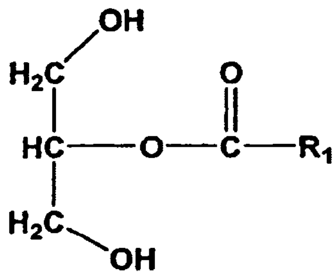

(B) au moins un diglycéride de formule (III) ou (IV) et

(III)

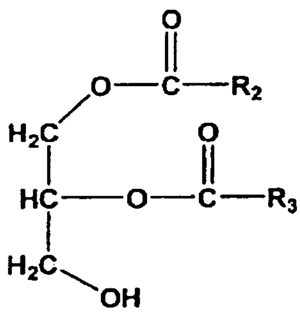

(IV)

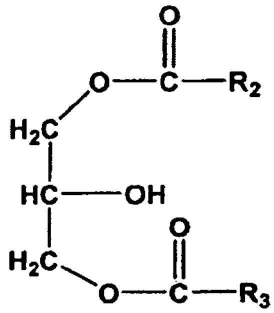

(D) au moins un triglycéride de formule (V)

V

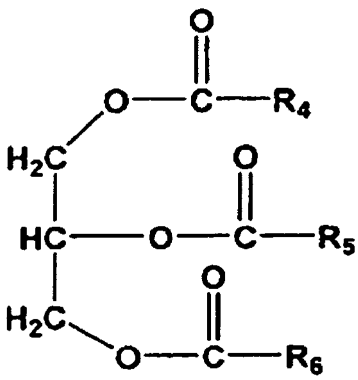

- où les radicaux $R_1$-CO-, $R_2$-CO-, $R_3$-CO-, $R_4$-CO-, $R_5$-CO- et $R_6$-CO- sont choisis, indépendamment l'un de l'autre, dans le groupe constitué par un radical acyle d'acide gras saturé, un radical acyle d'acide gras mono-insaturé à tétra-insaturé et un radical acyle d'acide gras au moins penta-insaturé,
- et où la somme des masses de tous les radicaux acyle d'acide gras au moins penta-insaturés présents dans le mélange, calculés sous forme d'acides libres, par rapport à la somme des masses de tous les radicaux acyle d'acide gras présents dans le mélange, à nouveau calculés sous forme d'acides gras libres, est d'au moins 40% en poids et d'au plus 80% en poids,
- et où la somme des masses de tous les radicaux acyle d'acide gras saturés présents dans le mélange, calculés sous forme d'acides libres, par rapport à la somme des masses de tous les radicaux acyle d'acide gras présents dans le mélange, à nouveau calculés sous forme d'acides gras libres, est d'au plus

$$\frac{85-a}{3}\% \; en \; poids$$

où a est la somme des masses de tous les radicaux acyle d'acide gras au moins penta-insaturés présents dans le mélange, calculés sous forme d'acides libres, par rapport à la somme des masses de tous les radicaux acyle d'acide gras présents dans le mélange, à nouveau calculés sous forme d'acides gras libres, en % en poids,
- et où la masse des radicaux acyle d'acide gras de l'acide stéarique, calculés sous forme d'acide gras libre, par rapport à la somme des masses de tous les radicaux acyle d'acide gras présents dans le mélange, à nouveau calculés sous forme d'acides gras libres, est d'au plus 2% en poids,
- et où la teneur en triglycérides par rapport à tous les glycérides des formules I-V est de 50 à 85% en poids et répond en outre à la condition selon laquelle la teneur en triglycérides par rapport à tous les glycérides des formules I-V est d'au moins

$$\frac{410-5a}{3}\% \; en \; poids$$

où a présente la signification définie ci-dessus,
- et où la teneur en diglycérides par rapport à tous les glycérides des formules I-V est d'au moins

$$100-3-b \,\% \; en \; poids$$

où b est la teneur en triglycérides par rapport à tous les glycérides des formules I-V en % en poids.

**2.** Utilisation du mélange selon la revendication 1 comme complément alimentaire ou additif alimentaire pour l'alimentation humaine ou comme fourrage pour l'alimentation des animaux.

**3.** Procédé pour la préparation du mélange selon la revendication 1 à partir d'un premier mélange contenant

(A) éventuellement au moins un monoglycéride de formule (I) ou (II)

(I)

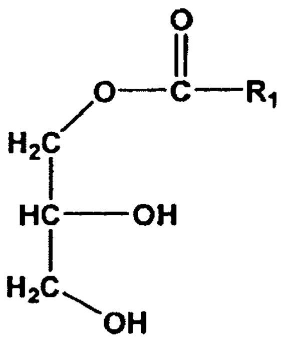

(II)

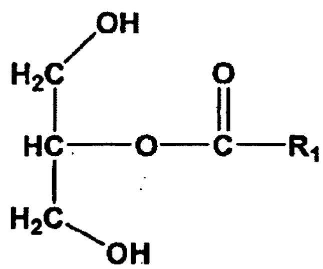

(B) éventuellement au moins un diglycéride de formule (III) ou (IV) et

(III)

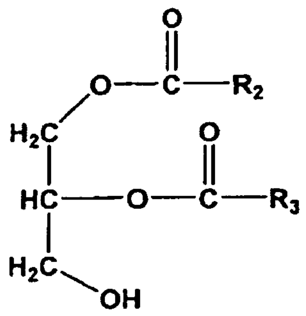

(IV)

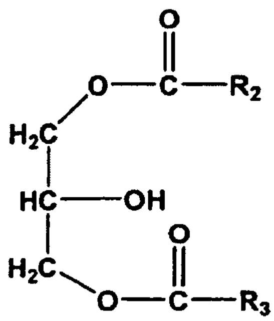

(D) au moins un triglycéride de formule (V)

(V)

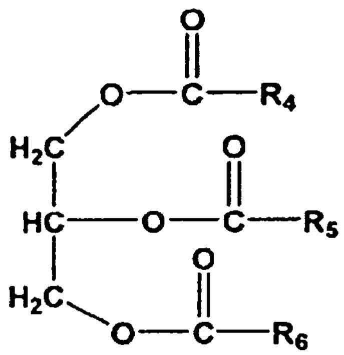

- où les radicaux $R_1$-CO-, $R_2$-CO-, $R_3$-CO-, $R_4$-CO-, $R_5$-CO- et $R_6$-CO- sont choisis, indépendamment l'un de l'autre, dans le groupe constitué par un radical acyle d'acide gras saturé, un radical acyle d'acide gras mono-insaturé à tétra-insaturé et un radical acyle d'acide gras au moins penta-insaturé,
- et où la somme des masses de tous les radicaux acyle d'acide gras au moins penta-insaturés présents dans le mélange, calculés sous forme d'acides libres, par rapport à la somme des masses de tous les radicaux acyle d'acide gras présents dans le mélange, à nouveau calculés sous forme d'acides gras libres, est supérieure au moins du facteur 1,3 à celle dans le premier mélange (facteur A),
- et où la somme des masses de tous les radicaux acyle d'acide gras saturés présents dans le mélange, calculés sous forme d'acides libres, par rapport à la somme des masses de tous les radicaux acyle d'acide gras présents dans le mélange, à nouveau calculés sous forme d'acides gras libres, est inférieure au moins du facteur 2 à celle dans le premier mélange (facteur B),
- et où la valeur du facteur A est inférieure à la valeur du facteur B,
- et où la teneur du premier mélange en triglycérides par rapport à tous les glycérides des formules I-V est d'au moins 90% en poids,
- et où le premier mélange contient des radicaux acyle d'acide gras au moins penta-insaturés et où, dans une forme de réalisation préférée de la présente invention, la somme des masses de tous les radicaux acyle d'acide gras au moins penta-insaturés présents dans le premier mélange, calculés sous forme d'acides libres, par rapport à la somme des masses de tous les radicaux acyle d'acide gras présents dans le premier mélange, à nouveau calculés sous forme d'acides gras libres, est d'au moins 15% en poids,
- et où le procédé comprend la transformation du premier mélange avec de l'eau ou avec un alcool comprenant 1 à 4 atomes de carbone en présence d'une lipase A de Candida antartica.

**4.** Procédé selon la revendication 3, où la transformation du premier mélange avec de l'eau ou avec un alcool comprenant 1 à 4 atomes de carbone est réalisée en présence d'une première lipase non régio-sélective présentant une spécificité élevée pour des acides gras saturés et une deuxième lipase non régio-sélective présentant une spécificité élevée pour des acides gras mono-insaturés par rapport à tous les acides gras insaturés, où la première

lipase est la lipase A de Candida antarctica et où la deuxième lipase est choisie parmi une des lipases de Candida rugosa ou de Candida cylindracea.

5. Procédé selon l'une quelconque des revendications 3 à 4, où le premier mélange est choisi dans le groupe constitué par une huile de poisson, une huile de crustacés marins, une huile de microalgues, une huile de microorganismes marins ou une huile de mammifères marins vivants, où on utilise de préférence une huile présentant une teneur supérieure à 20% en poids d'acides gras liés au moins penta-insaturés.

6. Procédé selon l'une quelconque des revendications 3 à 5, où la première lipase est utilisée sous forme libre ou immobilisée.

7. Procédé selon l'une quelconque des revendications 4 à 6, où la deuxième lipase est utilisée sous forme libre ou immobilisée.

8. Procédé selon l'une quelconque des revendications 3 à 7, où l'eau est ajoutée en une concentration de 2-50% en poids pour l'hydrolyse partielle et où l'éthanol est ajouté en une concentration de 5-50% en poids, ou un mélange d'eau et d'alcool à une concentration totale de 5-50% par rapport à la proportion d'huile étant ajouté.

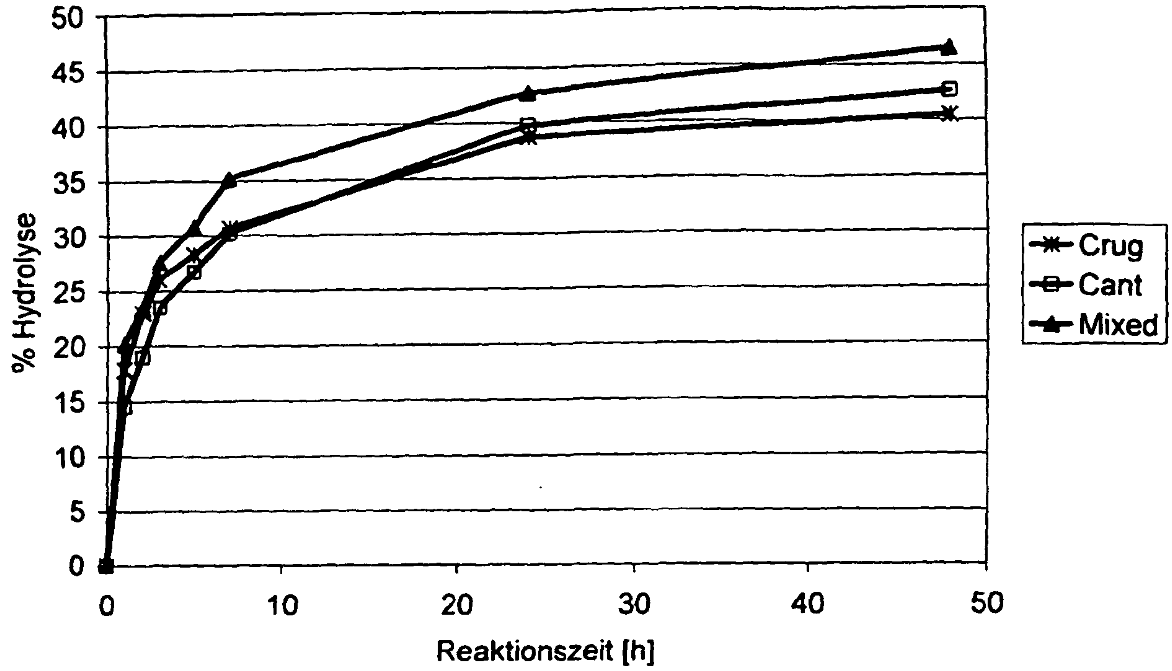
50
45
40
35
30
25
20
15
10
5
0
% Hydrolyse
0
10
20
30
40
50
Reaktionszeit [h]
Crug
Cant
Mixed

Fig. 1

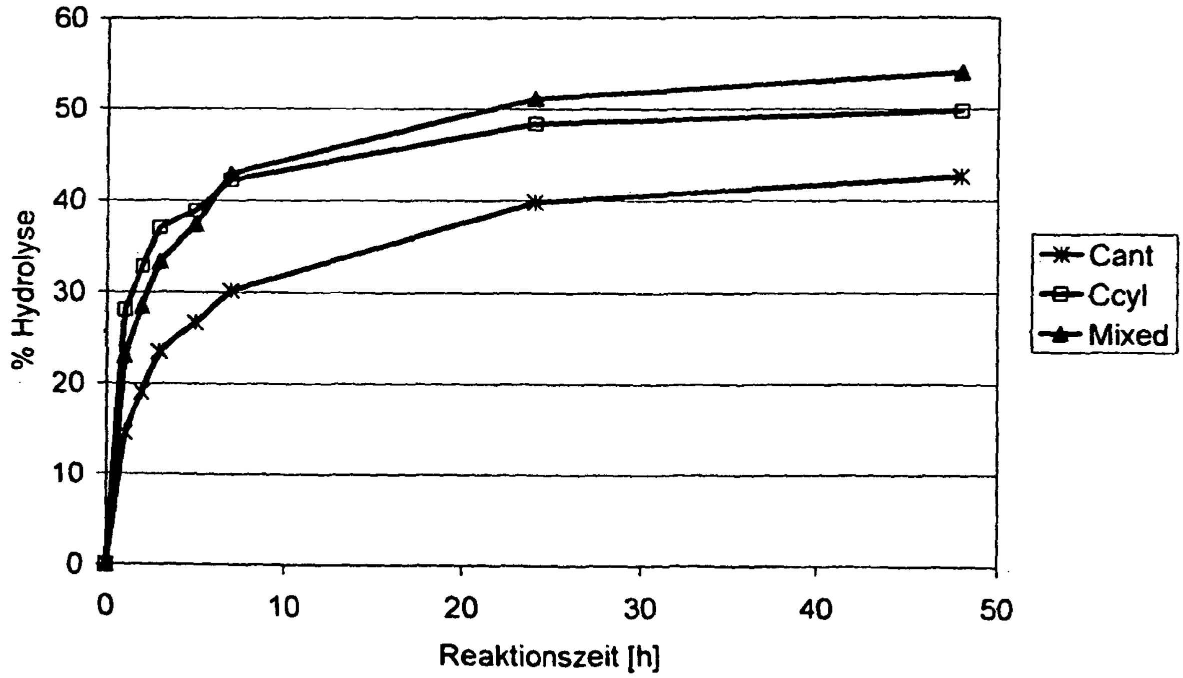
60
50
40
30
20
10
0
% Hydrolyse
0
10
20
30
40
50
Reaktionszeit [h]
Cant
Ccyl
Mixed

Fig. 2

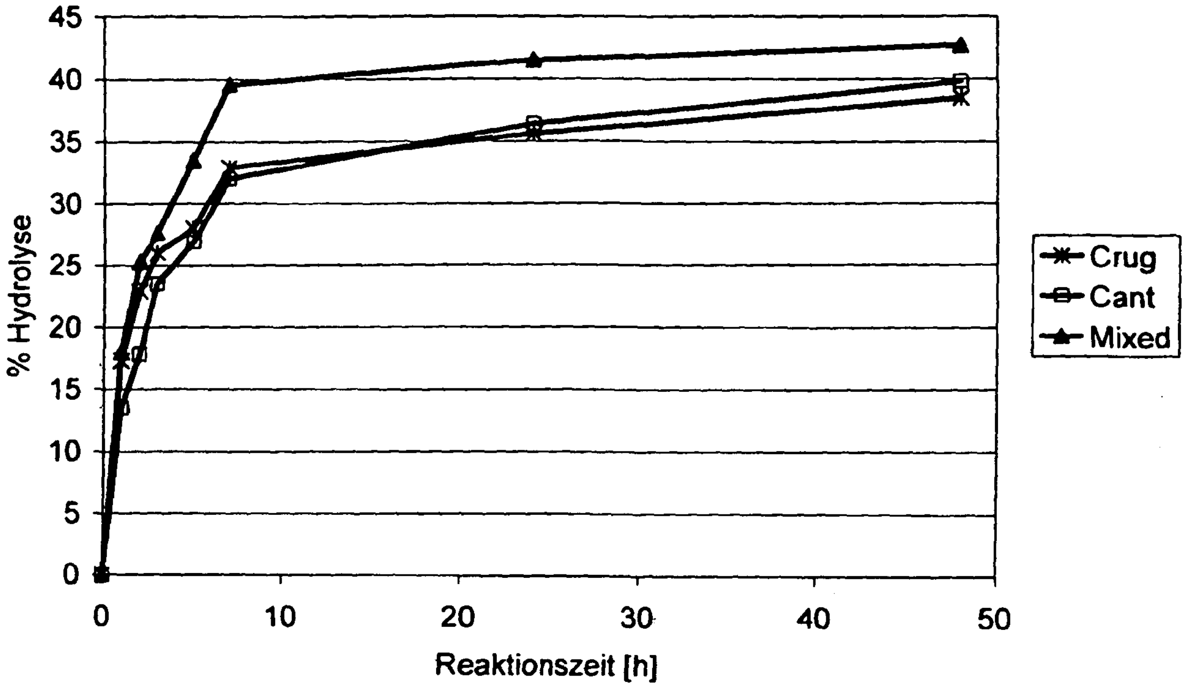
45
40
35
30
25
20
15
10
5
0
% Hydrolyse
0
10
20
30
40
50
Reaktionszeit [h]
Crug
Cant
Mixed

Fig. 3

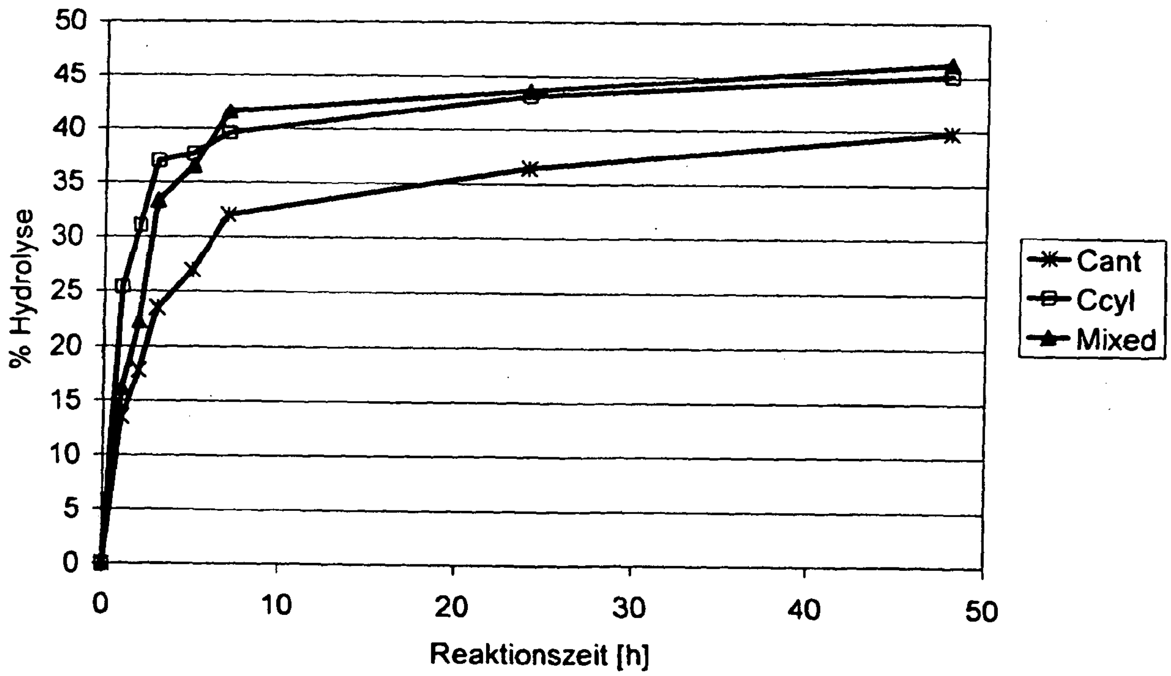
50
45
40
35
30
25
20
15
10
5
0
% Hydrolyse
0
10
20
30
40
50
Reaktionszeit [h]
Cant
Ccyl
Mixed

Fig. 4

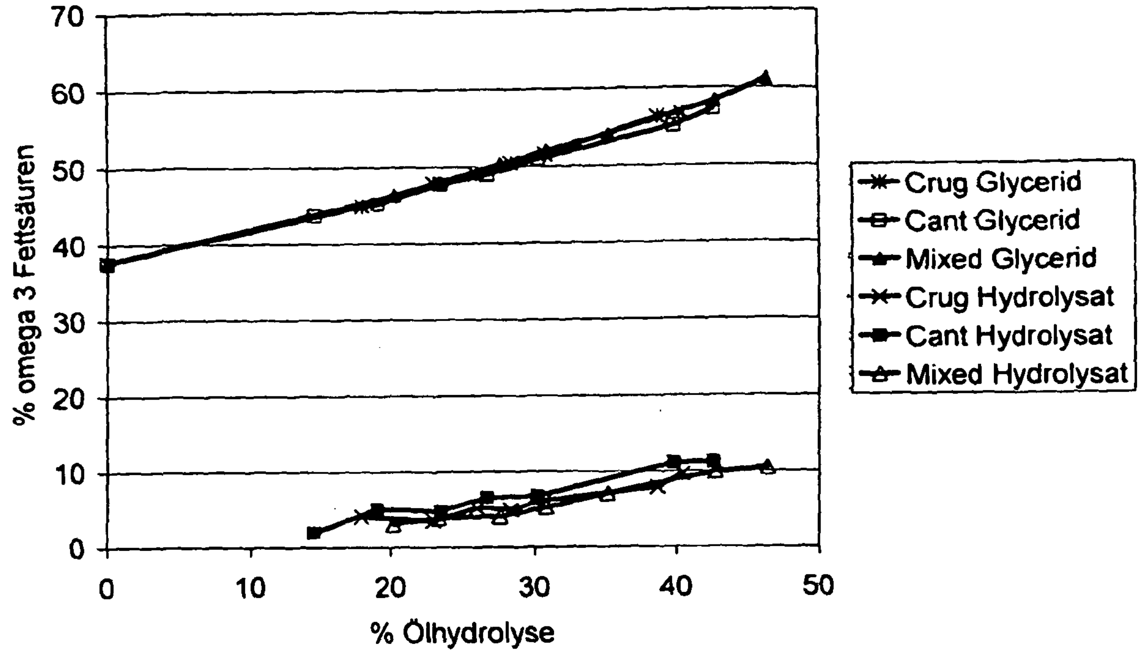
70
60
50
40
30
20
10
0
% omega 3 Fettsäuren
0
10
20
30
40
50
% Ölhydrolyse
Crug Glycerid
Cant Glycerid
Mixed Glycerid
Crug Hydrolysat
Cant Hydrolysat
Mixed Hydrolysat

**Fig. 5**

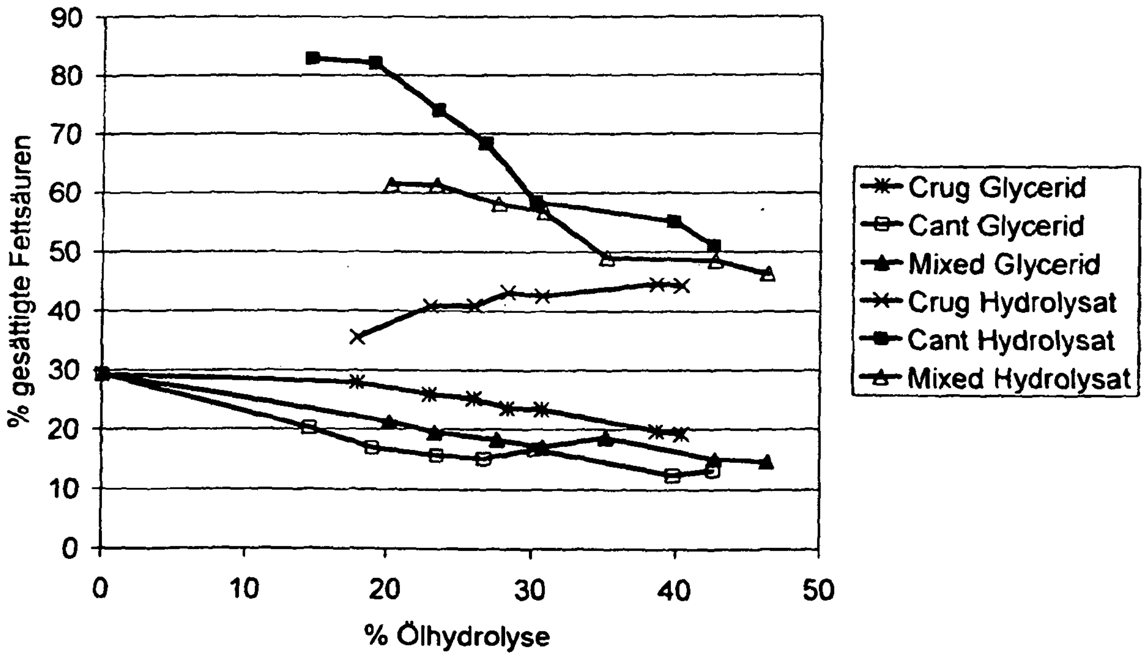
90
80
70
60
50
40
30
20
10
0
% gesättigte Fettsäuren
0
10
20
30
40
50
% Ölhydrolyse
Crug Glycerid
Cant Glycerid
Mixed Glycerid
Crug Hydrolysat
Cant Hydrolysat
Mixed Hydrolysat

**Fig. 6**

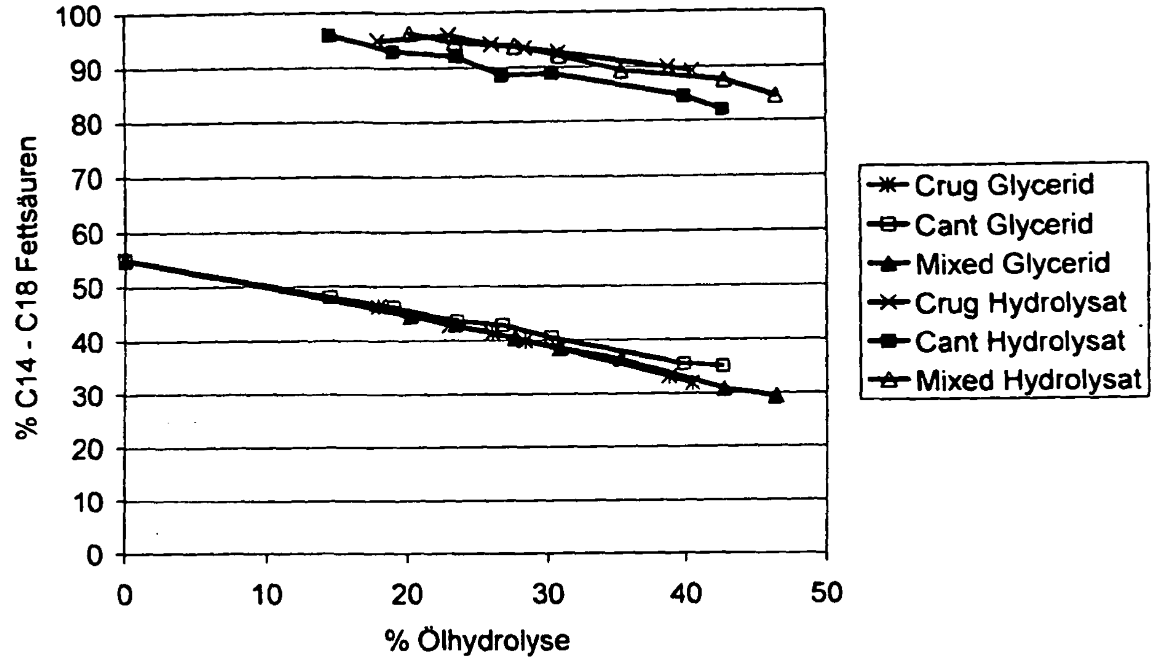
100
90
80
70
60
50
40
30
20
10
0
% C14 - C18 Fettsäuren
0
10
20
30
40
50
% Ölhydrolyse
Crug Glycerid
Cant Glycerid
Mixed Glycerid
Crug Hydrolysat
Cant Hydrolysat
Mixed Hydrolysat

Fig. 7

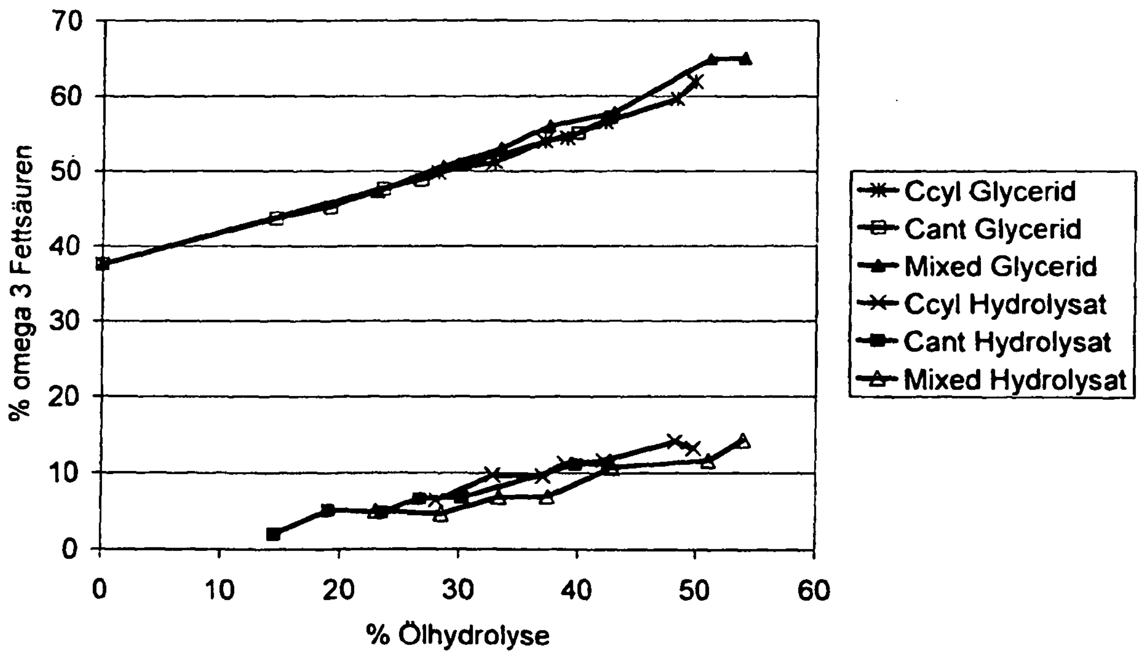
70
60
50
40
30
20
10
0
% omega 3 Fettsäuren
0
10
20
30
40
50
60
% Ölhydrolyse
Ccyl Glycerid
Cant Glycerid
Mixed Glycerid
Ccyl Hydrolysat
Cant Hydrolysat
Mixed Hydrolysat

Fig. 8

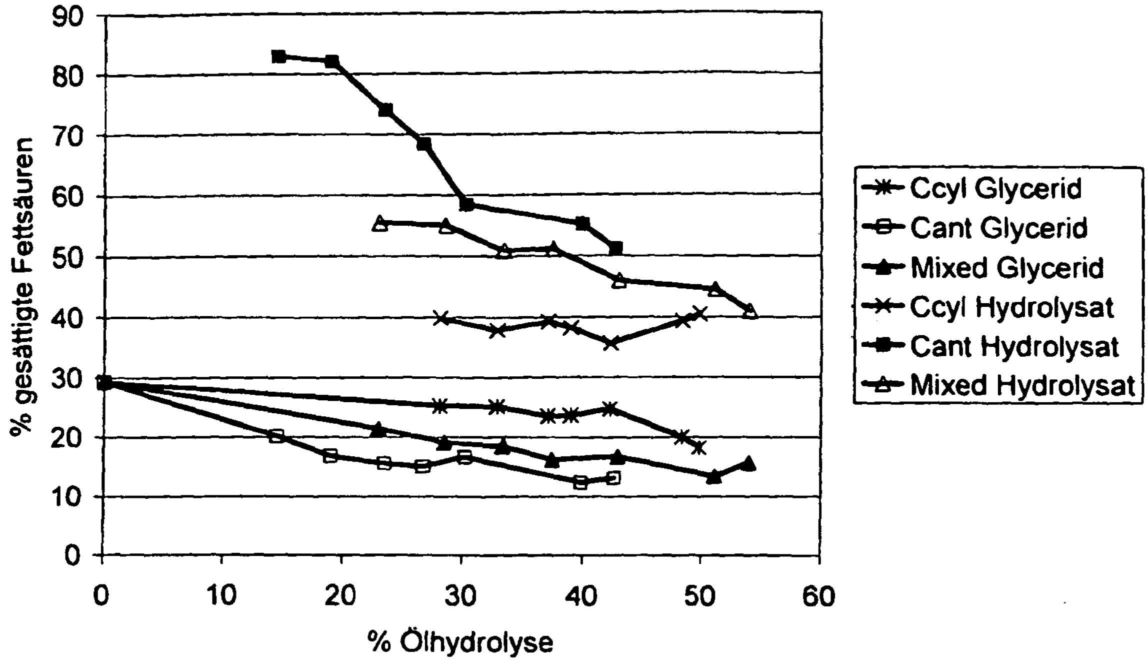
90
80
70
60
50
40
30
20
10
0
% gesättigte Fettsäuren
0
10
20
30
40
50
60
% Ölhydrolyse
Ccyl Glycerid
Cant Glycerid
Mixed Glycerid
Ccyl Hydrolysat
Cant Hydrolysat
Mixed Hydrolysat

**Fig. 9**

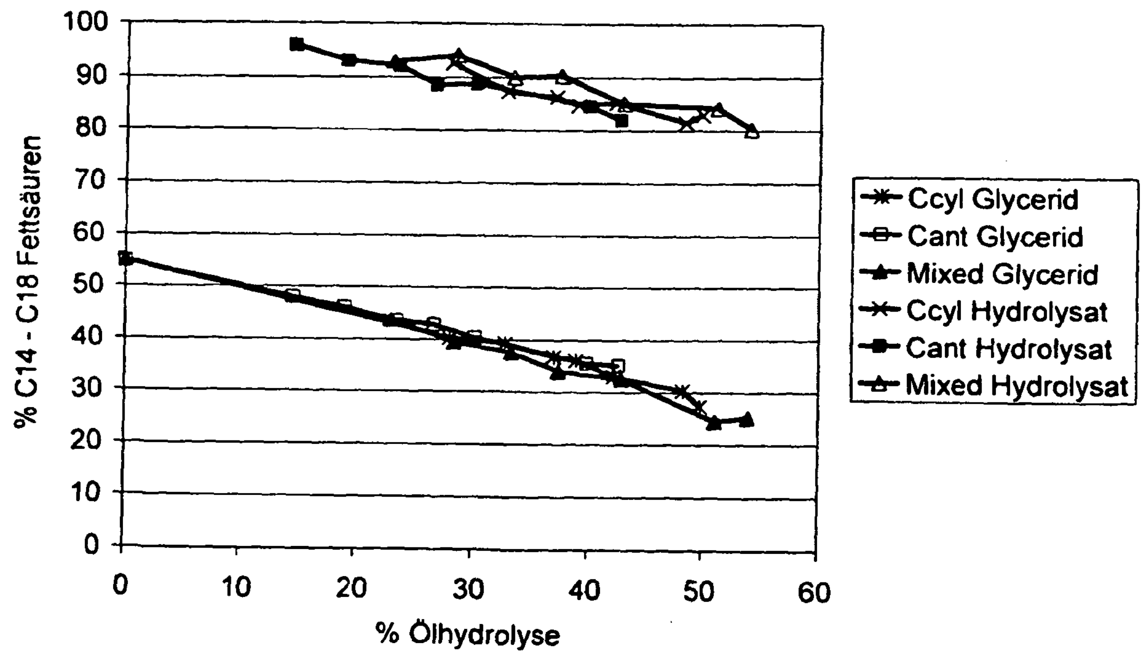
100
90
80
70
60
50
40
30
20
10
0
% C14 - C18 Fettsäuren
0
10
20
30
40
50
60
% Ölhydrolyse
Ccyl Glycerid
Cant Glycerid
Mixed Glycerid
Ccyl Hydrolysat
Cant Hydrolysat
Mixed Hydrolysat

**Fig. 10**

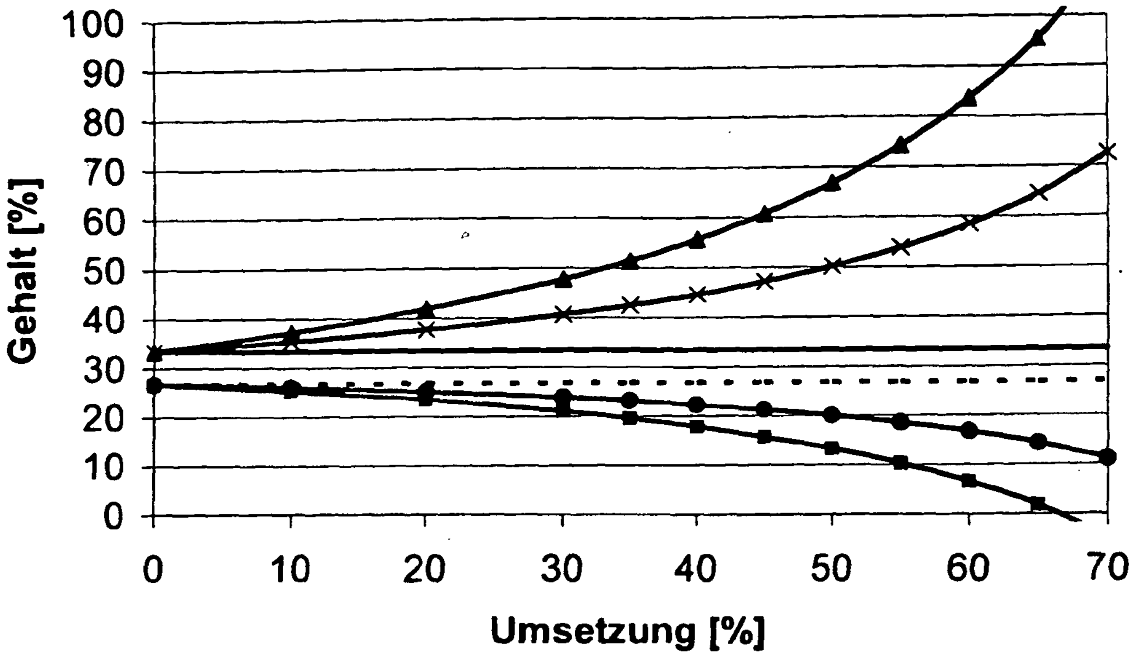
100
90
80
70
60
50
40
30
20
10
0
Gehalt [%]
0
10
20
30
40
50
60
70
Umsetzung [%]

Fig. 11

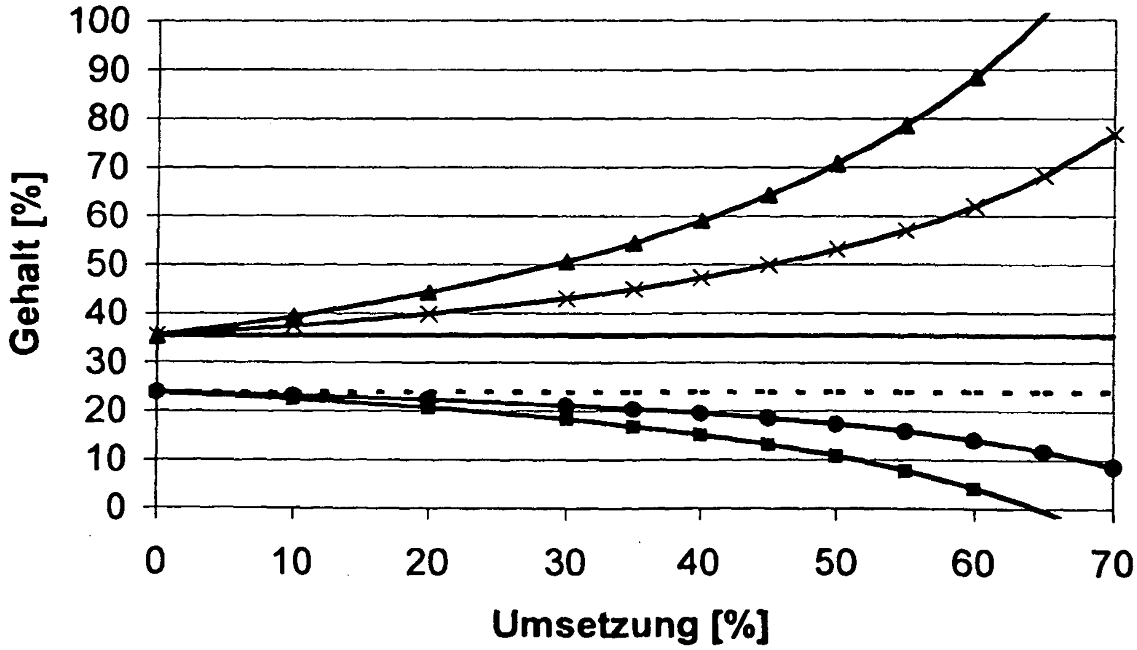
100
90
80
70
60
50
40
30
20
10
0
Gehalt [%]
0
10
20
30
40
50
60
70
Umsetzung [%]

Fig. 12

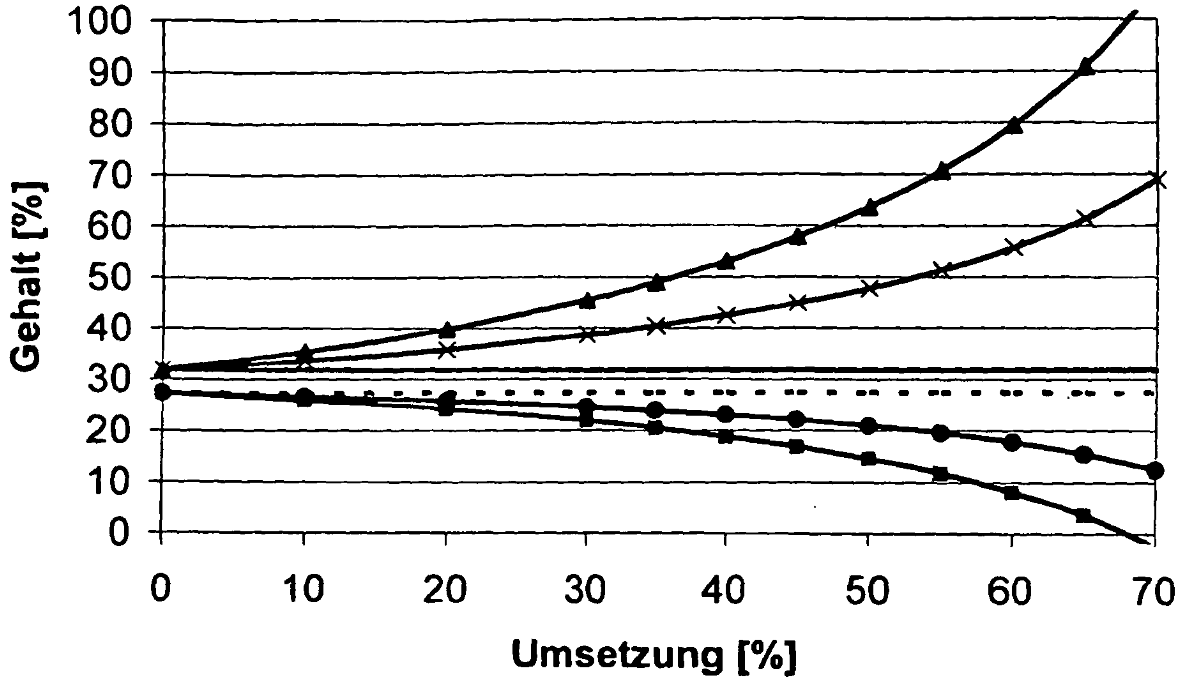
100
90
80
70
60
50
40
30
20
10
0
Gehalt [%]
0
10
20
30
40
50
60
70
Umsetzung [%]

Fig. 13

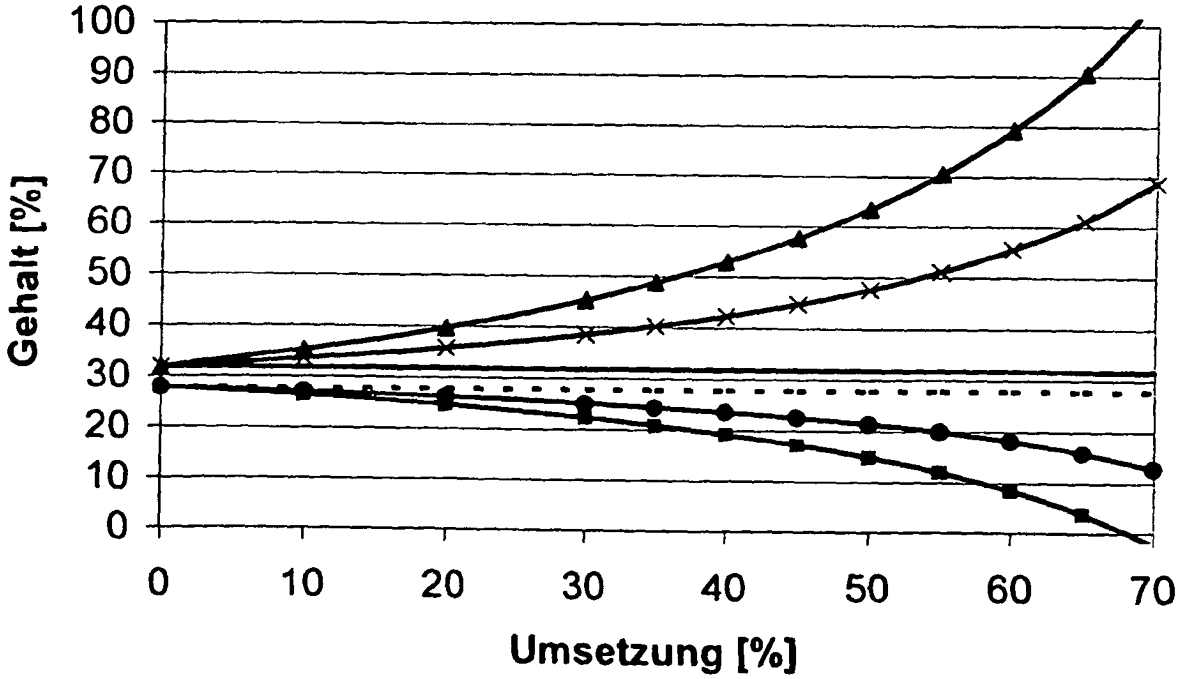
100
90
80
70
60
50
40
30
20
10
0
Gehalt [%]
0
10
20
30
40
50
60
70
Umsetzung [%]

Fig. 14

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0528844 A
- WO 9719601 A
- WO 9524459 A
- WO 9637586 A
- WO 9637587 A
- EP 0741183 A
- WO 9626287 A
- WO 0073254 A
- WO 04043894 A
- WO 0049117 A
- WO 9116443 A
- WO 8802775 A
- WO 03040091 A
- WO 07119811 A
- KR 100684642

### In der Beschreibung aufgeführte Nicht-Patentliteratur

- *J. Mol. Catal. B: Enzymatic,* 2005, vol. 37, 36-46
- *Biotechnology Advances,* 2006, vol. 24, 180-196
- **OKADA et al.** *Food chemistry,* 27. Marz 2007, vol. 103 (4), 1411-1419
- **YUKIHISA TANAKA et al.** *Journal of the American oil chemists society,* 01. Dezember 1992, vol. 69 (12), 1210-1214
- **WARWEL S. et al.** *Biotechnology letters,* 1999, vol. 21 (5), 431-436